(19) Europäisches Patentamt
European Patent Office
Office européen des brevets

(11) Publication number : **0 475 896 A1**

## (12) EUROPEAN PATENT APPLICATION

(21) Application number : **91810675.8**

(22) Date of filing : **23.08.91**

(51) Int. Cl.[5] : **C07D 498/18,** A61K 31/42, // (C07D498/18, 323:00, 307:00, 263:00)

(30) Priority : **31.08.90 US 576626**

(43) Date of publication of application : **18.03.92 Bulletin 92/12**

(84) Designated Contracting States : **BE CH DE FR GB IT LI NL SE**

(71) Applicant : **CIBA-GEIGY AG**
**Klybeckstrasse 141**
**CH-4002 Basel (CH)**

(72) Inventor : **Francis, John, Dr.**
**15 Wexford Way**
**Basking Ridge, NJ 07920 (US)**
Inventor : **Mugrage, Benjamin B., Dr.**
**10 Courter St.**
**Basking Ridge, N.J. 07920 (US)**

## (54) Polycyclic compounds.

(57) The invention relates to novel polycyclic derivatives of rifamycins of the formula

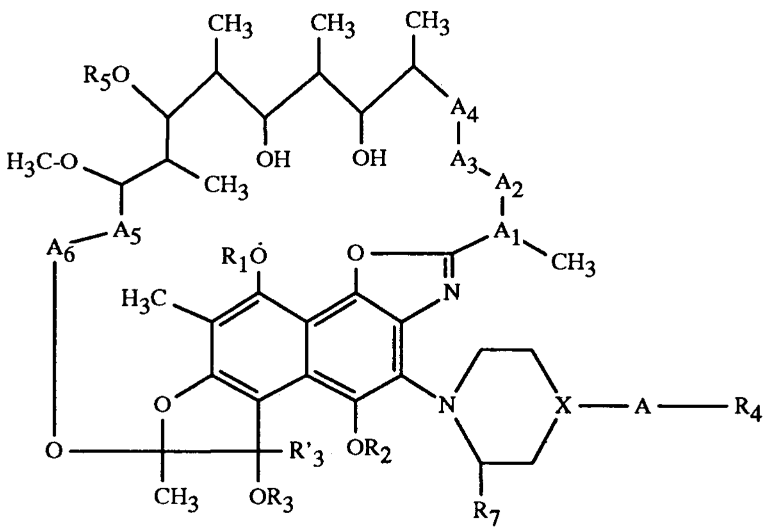

(I)

and the salts thereof, in which the structural elements $-A_1-A_2-$, $-A_3-A_4-$ and $-A_5-A_5-$ each denote ethylene or vinylene or the elements $-A_1-A_2$ and $-A_3-A_4-$ each denote ethylene and $-A_5-A_5-$ denotes vinylene ; X represents

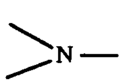

Or

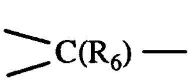

and $R_6$ denotes hydrogen or alkyl ; A denotes a bond, a bivalent aliphatic hydrocarbon radical or, if X represents

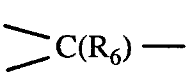 ,

EP 0 475 896 A1

represents the structural element $-X_1-A_1-$ in which $X_1$ denotes -O-, $-S(O)_n-$ or $-N(R_6)-$ n being 0, 1 or 2 and $R_6$ being hydrogen or alkyl and in which $A_1$ denotes a bivalent aliphatic hydrocarbon radical ; $R_1$ denotes hydrogen or acyl ; $R_2$ denotes acyl or an aliphatic radical ; and $R_3$ and $R_3'$ represent a common bond ; or $R_3$ denotes hydrogen, acyl or an aliphatic radical, and $R_3'$ is hydrogen or an aliphatic radical ; $R_4$ denotes optionally substituted bicycloheptyl, bicycloheptenyl or adamantyl ; $R_6$ denotes hydrogen or acetyl ;$R_7$ denotes hydrogen or alkyl ; the preparation and use thereof, and pharmaceutical products and the preparation thereof.

The invention relates to novel polycyclic derivatives of rifamycin SV of the formula

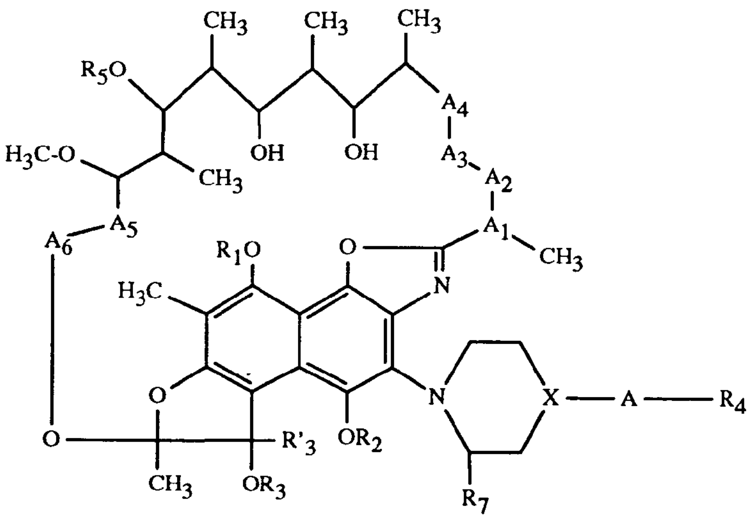

(I)

and the salts thereof, in which the structural elements $-A_1$ $-A_2$-, $-A_3$-$A_4$- and $-A_5$-$A_6$- each denote ethylene or vinylene or the elements $-A_1$-$A_2$- and $-A_3$-$A_4$- each denote ethylene and $-A_5$-$A_5$- denotes vinylene; X represents

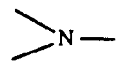

or

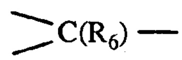

and $R_6$ denotes hydrogen or alkyl; A denotes a bond, a bivalent aliphatic hydrocarbon radical or, if X represents

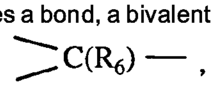,

represents the structural element $-X_1$-$A_1$- in which $X_1$ denotes -O-, $-S(O)_n$- or $-N(R_6)$- n being 0, 1 or 2 and $R_6$ being hydrogen or alkyl and in which $A_1$ denotes a bivalent aliphatic hydrocarbon radical; $R_1$ denotes hydrogen or acyl; $R_2$ denotes acyl or an aliphatic radical; and $R_3$ and $R_3'$ represent a common bond; or $R_3$ denotes hydrogen, acyl or an aliphatic radical, and $R_3'$ is hydrogen or an aliphatic radical; $R_4$ denotes optionally substituted bicycloheptyl, bicycloheptenyl or adamantyl; $R_6$ denotes hydrogen or acetyl; $R_7$ denotes hydrogen or alkyl; the preparation and use thereof, and pharmaceutical products and the preparation thereof.

The ring system numbering used as a basis essentially corresponds to that employed, for example, in US Patent No. 4,005,077.

The compounds of the formula I have several centres of chirality, and accordingly the present invention embraces the corresponding optical isomers, for example diastereoisomers.

A particular embodiment of the invention relates to derivatives of rifamycin SV of formula Ia

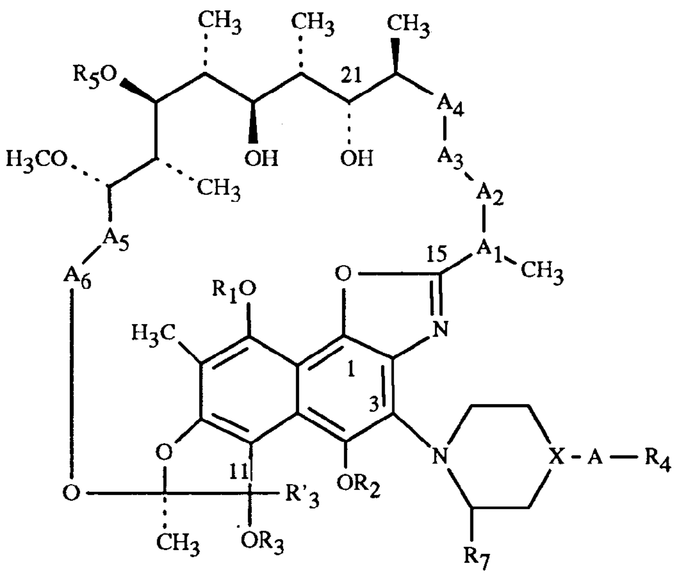

(Ia)

wherein symbols have meaning as defined for formula I above. The configurations at most C-atoms correspond to the known configurations of rifamycin S and rifamycin SV [cf. S. J. Danishefsky et al., J. Am. Chem. Soc. 109 (1987) 862-867]. If $-A_1-A_2-$ represents ethylene there is a further asymmetric centre at C- 16. The methyl group bonded to C- 16 is preferably in the α-position, i.e. below the plane of the paper like e.g. the 21 -hydroxy group. If $R_3$ denotes hydrogen or acyl and $R'_3$ is hydrogen, there is an additional asymmetric centre at C-11. In this case $R'_3$ may be in α- or β-position, but is preferably in α-position ($OR_3$ being β).

The compounds of the formula I or Ia can be in the form of salts, especially pharmaceutically utilizable (acceptable) salts. Because the compounds according to the invention have at least one basic centre, they are therefore able to form acid addition salts. The latter are formed, for example, with inorganic acids such as mineral acids, for example sulfuric acid, a phosphorus or hydrohalic acid, or with organic carboxylic acids such as optionally substituted, for example by halogen, $C_1$-$C_4$alkanecarboxylic acids, for example acetic acid, such as optionally unsaturated dicarboxylic acids, for example oxalic, malonic, succinic, maleic, fumaric, phthalic or terephthalic acid, such as hydroxy carboxylic acids, for example ascorbic, glycolic, lactic, mallic, tartaric or citric acid, such as amino acids, for example aspartic or glutamic acid, with a benzoic acid or with organic sulfonic acids such as optionally substituted, for example by halogen, $C_1$-$C_4$alkane- or arylsulfonic acids, for example methane-, bromobenzene- or p-toluenesulfonic acid. Appropriate acid addition salts can also be formed with an additional basic centre which is present where appropriate (for example

X = 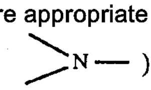 ).

Furthermore, the compounds according to the invention which have an acidic phenolic hydroxyl group may form salts ($R_1$= H) with bases, for example alkali metals, such as sodium or potassium salts. In addition, corresponding inner salts can be formed. Also embraced are salts unsuitable for pharmaceutical uses, because the latter can be employed, for example, for the isolation or purification of compounds according to the invention or the pharmaceutically utilizable salts thereof.

A bivalent aliphatic hydrocarbon radical denotes, in particular, alkylene, alkenylene and alkynylene, where the multiple bond is located in a position higher than α to the piperazine nitrogen atom

(X = 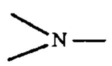 ).

Acyl is derived, for example, from an organic carboxylic acid, a substituted carbonic acid or an organic sulfonic acid. Examples of such radicals are alkanoyl which is optionally substituted by a radical selected from esterified, amidated carboxy, halogeno, and aryl, or are carbocyclic or heterocyclic aroyl, alkoxycarbonyl, arylalkoxycarbonyl, amino-carbonyl which is optionally mono- or disubstituted by alkyl, or are alkanesulfonyl, halogenoalkanesulfonyl, arylalkanesulfonyl, cycloalkanesulfonyl or arylsulfonyl. Esterified carboxy, for example, represents alkoxycarbonyl or alkoxy-alkoxycarbonyl and amidated carboxy represents, for example, carbamoyl optionally mono- or di-substituted by a substituent selected from alkyl and phenylalkyl. Preferably the corresponding substituent of alkanoyl is attached to position higher than the α position. In this connection, aryl denotes in each case, in particular, phenyl or naphthyl and carbocyclic aroyl, in particular benzoyl or

naphthoyl, and heterocyclic aroyl, in particular 5- or 6-membered monocyclic monoaza-, monooxa- or monothiaaroyl, such as pyrroloyl, pyridoyl, furoyl or thenoyl, where such aryl or aroyl radicals are unsubstituted or substituted one or more times, for example two or three times, for example by a substituent selected from the group consisting of halogen, alkyl, alkoxy, hydroxyl, alkanoyloxy, trifluoromethyl and nitro. Acyl also includes (pyrrolidino-, piperidino-, homopiperidino-, morpholino-, thiomorpholino-)carbonyl, furthermore adamantylcarbonyl, biphenylylcarbonyl, and bicycloheptylcarbonyl. $R_1$ preferably is pivaloyl, furthermore carbamoyl which is optionally mono- or di-substituted by $C_1$-$C_4$-akyl, such as N,N-dimethylcarbamoyl.

An aliphatic radical represents, for example, alkyl or and also alkenyl, which is optionally substituted by a radical selected from cycloalkyl, aryl, optionally etherified hydroxyl, optionally substituted amino, optionally esterified and amidated carboxy. Aryl represents, for example, phenyl or furthermore naphthyl which may be unsubstituted or substituted one or more times, for example, by a substituent selected from the group consisting of halogen, alkyl, alkoxy, hydroxyl, alkanoyloxy, trifluoromethyl and nitro. Etherified hydroxyl represents, for example, alkoxy, hydroxy-alkoxy, alkoxy-alkoxy, phenyl-alkoxy, phenyl-alkoxy-alkoxy, whereas substituted amino represents, for example, amino which is optionally mono- or di-substituted by a substituent selected from alkyl, phenyl-alkyl and phenyl. Esterified carboxy represents, for example, alkoxycarbonyl, phenyl-alkoxycarbonyl or alkoxy-alkoxycarbonyl, and amidated carboxy represents, for example, carbamoyl which is unsubstituted or mono- or di-substituted by a substituent selected from alkyl, phenyl-alkyl and phenyl.

An aromatic radical is, if not defined otherwise, in particular, phenyl, furthermore naphthyl, which are unsubstituted or substituted, for example, one or more times, such as two or three times, for example, by a substituent selected from the group consisting of halogen, alkyl, alkoxy, hydroxyl, alkanoyloxy, trifluoromethyl and nitro.

The general definitions used hereinbefore and hereinafter have primarily the following meanings, unless defined differently:

The expression "lower" means that corresponding groups and compounds in each case contain in particular not more than 7, preferably not more than 4, carbon atoms.

Alkyl denotes, in particular, $C_1$-$C_7$alkyl and is, for example, methyl, ethyl, propyl, isopropyl, n-butyl, isobutyl, sec-butyl, tert-butyl and furthermore embraces appropriate pentyl, hexyl and heptyl radicals. $C_1$-$C_4$Alkyl is preferred.

Bicycloheptyl which may be substituted, for example, mono- or disubstituted, e.g. by alkyl, in particular by $C_1$-$C_7$alkyl, represents, for example, bicyclo[2.2.1]heptyl, such as bornyl, neobornyl, isobornyl, norbornyl, e.g. 2-norbornyl, or bicyclo[3.1.1]heptyl, such as bicyclo[3.1.1]hept-2-yl or 6,6-dimethyl-bicyclo[3.1.1] heptyl. The term bornyl is synonymous with bornanyl.

Bicycloheptenyl which may be substituted, for example, mono- or disubstitued, e.g. by alkyl, in particular by $C_1$-$C_7$alkyl, represents, for example, bicyclo[2.2. 1]heptenyl, such as norborn-5-en-2-yl, or bicyclo[3.1.1]heptenyl, such as 6,6-dimethyl-bicyclo[3.1.1]hept-2-en-2-yl.

Adamantyl which may be substituted, for example, mono- or disubstituted, e.g. by alkyl, in particular by $C_1$-$C_7$alkyl, preferably represents 1-adamantyl, furthermore 2-adamantyl.

Alkylene denotes, in particular, $C_1$-$C_7$alkylene and is straight-chain or branched and denotes, for example, methylene, ethylene, propylene and butylene as well as 1,2-propylene, 2-methyl-1,3-propylene or 2,2-dimethyl-1,3-propylene. $C_1$-$C_4$Alkylene is preferred, primarily methylene.

Alkenylene denotes, in particular, $C_3$-$C_7$alkenylene and is straight-chain or branched and denotes, for example, 1,3-prop-2-enylene, 1,4-but-2-, 1,4-but-3-enylene, 1,3-but-2-enylene, 2,4-but-3-enylene, 1,5-pent-2-, -3-, -4-enylene, furthermore appropriate hexenylene and heptenylene radicals. $C_3$-$C_5$Alkenylene is preferred.

Alkynylene denotes, in particular, $C_3$-$C_7$alkynylene and is straight-chain or branched and denotes, for example, 1,3-prop-2-ynylene, 1,4-but-2-, 1,4-but-3-ynylene, 1,5-pent-2-, -3- and -5-ynylene, furthermore appropriate hexynylene and heptynylene radicals. $C_3$-$C_5$Alkynylene is preferred.

Alkanoyl denotes, in particular, $C_2$-$C_8$alkanoyl and is, for example, acetyl, propionyl, butyryl, isobutyryl or pivaloyl. Preferred, especially for $R_1$, is branched $C_2$-$C_6$alkanoyl, primarily pivaloyl, whereas $R_2$ and $R_3$ in particular are represented by $C_2$-$C_6$alkanoyl, primarily acetyl or propionyl.

Halogen is, in particular, halogen with atomic number up to and including 35, such as fluorine, chlorine and bromine, furthermore iodine.

Alkoxy denotes, in particular, $C_1$-$C_7$alkoxy and is, for example, methoxy, ethoxy, propyloxy, isopropyloxy, n-butyloxy, isobutyloxy and tert-butyloxy. $C_1$-$C_4$Alkoxy is preferred.

Alkoxycarbonyl is, in particular, $C_2$-$C_8$alkoxycarbonyl and is, for example, methoxy-, ethoxy-, propyloxy- or pivaloyloxy-carbonyl. $C_2$-$C_5$Alkoxycarbonyl is preferred.

Alkanesulfonyl is, in particular, $C_1$-$C_7$alkanesulfonyl and is, for example, methane-, ethane-, n-propane- or isopropanesulfonyl. $C_1$-$C_4$Alkanesulfonyl is preferred.

Halogenalkanesulfonyl is, in particular, halo-$C_1$-$C_7$alkanesulfonyl, in particular halo-$C_1$-$C_4$alkylsulfonyl, and is, for example, trifluoromethane-, difluoromethane- or 1,1,2-trifluoroethanesulfonyl.

Cycloalkyl denotes, in particular, $C_3$-$C_7$cycloalkyl and denotes, for example, cyclopropyl, -butyl, -pentyl, -hexyl or -heptyl. Cyclopentyl and cyclohexyl is preferred.

Cycloalkanesulphonyl is, in particular, $C_3$-$C_6$cyclo-$C_1$-$C_4$alkanesulphonyl, such as cyclopropyl-, cyclopentyl- or cyclohexyl(m)ethanesulphonyl.

Alkoxyalkoxycarbonyl is, in particular, $C_1$-$C_4$alkoxy-$C_1$-$C_4$alkoxycarbonyl, preferably ethoxyethoxycarbonyl, methoxyethoxycarbonyl and isopropyloxyethoxycarbonyl.

Phenylalkyl is, in particular, phenyl-$C_1$-$C_7$alkyl, such as benzyl, 2-phenylethyl or 3-phenylpropyl. Phenyl-$C_1$-$C_4$alkyl is preferred.

Naphthyl is 1- or 2-naphthyl.

Pyrroloyl is, for example, 2- or 3-pyrroloyl. Furoyl is 2- or 3-furoyl and thenoyl is 2- or 3-thenoyl, while suitable pyridoyl is 2-, 3- and 4-pyridoyl.

Alkanoyloxy is, in particular, $C_2$-$C_8$alkanoyloxy, in particular, $C_2$-$C_5$alkanoyloxy, such as acetyloxy, propionyloxy or pivaloyoxy.

Alkenyl is, in particular, $C_3$-$C_7$alkenyl and is, for example, 2-propenyl or 1-, 2- or 3-butenyl. $C_3$-$C_5$Alkenyl is preferred.

Hydroxyalkoxy is, in particular hydroxy-$C_1$-$C_7$-alkoxy, especially $C_1$-$C_4$-alkoxy, such as hydroxymethoxy, 2-hydroxyethoxy or 3-hydroxypropyloxy.

Alkoxyalkoxy is, in particular, $C_1$-$C_4$alkoxy-$C_1$-$C_4$alkoxy, such as 2-methoxyethoxy, 2-ethoxyethoxy, 2-n-propyloxyethoxy or ethoxymethoxy.

Phenylalkoxy is, in particular, phenyl-$C_1$-$C_4$alkoxy, such as benzyloxy, 1- or 2-phenylethoxy, or 1-, 2- or 3-phenylpropyloxy.

Phenylalkoxyalkoxy is, in particular, phenyl-$C_1$-$C_4$alkoxy-$C_1$-$C_4$alkoxy, such as 2-benyzyloxyethoxy, 2-(2-phenylethyl)-ethoxy.

Phenylalkoxycarbonyl is, in particular, phenyl-$C_1$-$C_4$alkoxycarbonyl, such as benzyloxycarbonyl or 2-phenyloxycarbonyl.

It is known of derivatives which are derived, for example, from rifamycin SV that they have pronounced antibiotic properties and can be employed, for example, for the treatment of tuberculosis. All the more important is the experimentally verified finding that the compounds of the formula I and Ia and the pharmaceutically utilizable salts thereof show no corresponding antibiotic activity in the customary pharmacological test models.

On the other hand, surprisingly they have a significant lipid-lowering action which can be detected in animal experiments, preferably on mammals, for example on rats. Thus, the lowering of very low density, low density and high density lipoproteins (VLDL, LDL and HDL respectively) in the serum can be shown in two designs of test, namely in genetically hypercholesterolaemic male rats (design A) and normolipaemic rats of both sexes (design B).

Albino rats (Sprague-Dawley derivatives of the strain Tif:RAI) with a body weight of 180-240 g, which have free access to standard rat feed and drinking water, are used. The test compound is administered in a supplemented maize starch solution (3% aqueous maize starch with 0.33 % Tween 80 and 5 % polyethylene glycol solution with a mean molecular weight of 400) orally to groups of 6 rats each day for 5 consecutive days. The animals are sacrificed two hours after the last dose. The animals receive no more food for 16 hours before their death. The blood is collected in a 0.05 % strength aqueous ethylenediaminetetraacetic acid solution. After centrifugation until the blood cells have sedimented, the content of cholesterol and triglycerides is analysed enzymatically using, for example, the test systems supplied by Sigma Chemical Co. (St. Louis, MO, USA). For the HDL-cholesterol determination, a solution of heparin and manganese chloride (final concentration = 1.3 g/l or 46 mMol) is added to 0.5 ml of EDTA-plasma. The precipitate which forms is sedimented by centrifugation, and the cholesterol concentration in the supernatant is analysed enzymatically as for the total cholesterol. The difference between the latter value and the cholesterol value determined directly on an aliquot of the complete serum is, according to Warnick, G.R. et al., J. Lipid Res. 19; 65-76 (1978), equivalent to the VLDL- and LDL-cholesterol.

The testing for an antibiotic reaction is carried out, for example, on the one hand in vitro by determining the mean effective concentration $EC_{50}$ for the inhibition of RNA polymerase of Escherichia coli, and the minimum inhibitory concentration (MIC) in a conventional plate test, and on the other hand in vivo on infected mice and rats by determining the $ED_{50}$ (effective dose which keeps 50 % of the experimental animals alive). The microorganisms used for the present purpose are, in particular, Mycobacterium tuberculosis TB $H_{37}$Rv and Staphylococcus aureus. In the case of compounds with a lipid-lowering indication, an antibiotic activity is regarded as disadvantageous because it may lead, especially on long-term administration, to the development of strains of microorganisms resistant to antibiotics.

In the test methods described above, the compounds according to the invention display a significant hypolipidaemic activity on repeated administration in the dose range from about 0.1 to about 50 mg/kg/day; by contrast, they have negligible antibiotic activity in the abovementioned tests.

Thus, for example, it is possible to show, depending on the experimental designs, that the minimum effective dose of the compounds according to the invention is about 0.1 to about 10 mg/kg on single administration, and a 50-70 % lowering of total cholesterol can be achieved by repeated administration of 30 mg/kg a day. Moreover, the compounds have virtually no antibiotic activity; an $EC_{50}$ for inhibition of RNA polymerase is not yet reached at 100 μg/ml, and the MIC for various pathogenic strains of Staphylococcus aureus is above 130 μg/ml. Such values are about 1000 times higher than concentrations normally necessary for a corresponding effect. Also in vivo, using mice infected with Staphylococcus aureus, the compound proves to have no antibiotic activity at a single dose of 200 mg/kg.

Thanks to their LDL-lowering action, the compounds according to the invention can be used, for example, as hypolipidaemics for the treatment of hyperlipidaemias, principally of types IIa and IIb, and arteriosclerosis, for example when hyperlipoproteinaemia is present as risk factor.

Accordingly, the compounds of the formula I and Ia and the pharmaceutically utilizable salts thereof can be used, for example, as pharmaceuticals, for example as hypolipidaemics for the treatement of hyperlipidaemias, principally of types IIa and IIb, and of arteriosclerosis when hyperlipoproteinaemia is present as risk factor. The invention furthermore relates to the use of the compounds according to the invention for the preparation of medicaments, in particular of hypolipidaemics and antiarteriosclerotics, and for therapeutic and prophylactic treatment. Also included therein is the industrial preparation of the active substances.

Furthermore, the compounds according to the present invention may be used as starting material.

The invention relates in particular to compounds of the formula I and Ia and their salts, in which the structural elements $-A_1-A_2-$, $-A_3-A_4-$ and $-A_5-A_6-$ have the meanings given; X represents

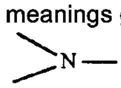

or

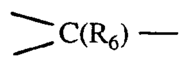

and $R_6$ denotes hydrogen or alkyl; A denotes a bond, alkylene, alkenylene or alkynylene or, if X represents

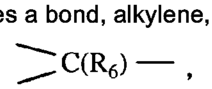 ,

represents the structural element $-X_1-A_1-$ in which $X_1$ denotes -O-, $S(O)_n$- or $-N(R_6)-$ n being 0, 1 or 2 and $R_6$ being hydrogen or alkyl and in which $A_1$ denotes alkylene, alkenylene or alkynylene; $R_1$ denotes hydrogen, alkanoyl which is optionally substituted by a radical selected from esterified, amidated carboxy, halogeno, and aryl, or denotes carbocyclic or heterocyclic aroyl, alkoxycarbonyl, arylalkoxycarbonyl, amino-carbonyl which is optionally mono- or disubstituted by alkyl, or denotes alkanesulfonyl, halogenoalkanesulfonyl, arylalkanesulfonyl, cycloalkanesulfonyl or arylsulfonyl or denotes (pyrrolidino-, piperidino-, homopiperidino-, morpholino-, thiomorpholino-)carbonyl, furthermore adamantylcarbonyl, biphenylylcarbonyl or bicycloheptylcarbonyl; $R_2$ denotes alkanoyl which is optionally substituted by a radical selected from esterified, amidated carboxy, halogeno, and aryl, or denotes carbocyclic or heterocyclic aroyl, alkoxycarbonyl, arylalkoxycarbonyl, aminocarbonyl which is optionally mono- or disubstituted by alkyl, or denotes alkanesulfonyl, halogenoalkanesulfonyl, arylalkanesulfonyl, cycloalkanesulfonyl or arylsulfonyl or denotes alkyl and also alkenyl, which are optionally substituted by a radical selected from the group cycloalkyl, aryl, optionally etherified hydroxyl, optionally substituted amino, optionally esterified and amidated carboxy;

and $R_3$ and $R_3'$ represent a common bond; or $R_3$ denotes hydrogen, alkanoyl which is optionally substituted by a radical selected from esterified, amidated carboxy, halogeno, and aryl, or denotes carbocyclic or heterocyclic aroyl, alkoxycarbonyl, arylalkoxycarbonyl, amino-carbonyl which is optionally mono- or disubstituted by alkyl, or denotes alkanesulfonyl, halogenoalkanesulfonyl, arylalkanesulfonyl, cycloalkanesulfonyl or arylsulfonyl or denotes alkyl and also alkenyl, which are optionally substituted by a radical selected from the group cycloalkyl, aryl, optionally etherified hydroxyl, optionally substituted amino, optionally esterified and amidated carboxy; and $R_3'$ is hydrogen or alkyl and also alkenyl, which are optionally substituted by a radical selected from the group cycloalkyl, aryl, optionally etherified hydroxyl, optionally substituted amino, optionally esterified and amidated carboxy; $R_4$ denotes bicycloheptyl, bicycloheptyl or adamantyl which in each case may be substituted by alkyl; $R_5$ denotes hydrogen or acetyl; $R_7$ denotes hydrogen or alkyl; where the (hetero-)aromatic radicals in each case are unsubstituted or substituted one or more times by a substituent selected from the group consisting of halogen, alkyl, alkoxy, hydroxyl, alkanoyloxy, trifluoromethyl and nitro.

The invention relates in particular to compounds of the formula I and Ia and their salts, in which the structural

elements $-A_1-A_2-$, $-A_3-A_4-$ and $-A_5-A_6-$ have the meanings given; X represents

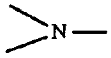

or

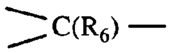

and $R_6$ denotes hydrogen or $C_1$-$C_7$alkyl; A denotes denotes a bond, $C_1$-$C_7$alkylene, $C_3$-$C_7$alkenylene or $C_3$-$C_7$alkynylene or, if X represents

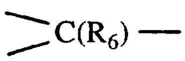 ,

represents the structural element $-X_1-A_1-$ in which $X_1$ denotes -O-, $S(O)_n$- or $-N(R_6)-$ n being 0, 1 or 2 and $R_6$ being hydrogen or $C_1$-$C_7$alkyl and in which $A_1$ denotes $C_1$-$C_7$alkylene, $C_3$-$C_7$alkenylene or $C_3$-$C_7$alkynylene; $R_1$ denotes hydrogen, $C_2$-$C_8$alkanoyl, carboxy-$C_2$-$C_8$alkanoyl, $C_1$-$C_7$alkoxycarbonyl- or $C_1$-$C_7$alkoxy-$C_1$-$C_7$alkoxycarbonyl-$C_2$-$C_8$alkanoyl, carbamoyl-$C_2$-$C_8$alkanoyl, in which carbamoyl is optionally mono- or disubstituted by a radical selected from $C_1$-$C_7$alkyl and phenyl-$C_1$-$C_7$alkyl, denotes halogeno-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or denotes $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkanesulfonyl or benzene- or naphthylsulfonyl, or denotes (pyrrolidino-, piperidino-, homopiperidino-, morpholino-, thiomorpholino-)carbonyl, furthermore adamantylcarbonyl, biphenylylcarbonyl or bicycloheptylcarbonyl;

$R_2$ denotes $C_2$-$C_8$alkanoyl, carboxy-$C_2$-$C_8$alkanoyl, $C_1$-$C_7$alkoxycarbonyl- or $C_1$-$C_7$alkoxy-$C_1$-$C_7$alkoxy-carbonyl-$C_2$-$C_8$alkanoyl, carbamoyl-$C_2$-$C_8$alkanoyl, in which carbamoyl is optionally mono- or disubstituted by a radical selected from $C_1$-$C_7$alkyl and phenyl-$C_1$-$C_7$alkyl, denotes halogeno-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or denotes $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkanesulfonyl or benzene- or naphthylsulfonyl, or denotes $C_1$-$C_7$alkyl or $C_3$-$C_7$alkenyl which are optionally substituted by a radical selected from $C_3$-$C_7$cycloalkyl, phenyl, naphthyl, hydroxyl, $C_1$-$C_7$alkoxy, hydroxy-$C_1$-$C_7$alkoxy, $C_1$-$C_7$alkoxy-$C_1$-$C_7$alkoxy, phenyl-$C_1$-$C_7$alkoxy, phenyl-$C_1$-$C_7$alkoxy-$C_1$-$C_7$alkoxy, amino which is optionally substituted by a substituent selected from $C_1$-$C_7$alkyl, phenyl-$C_1$-$C_7$alkyl and phenyl, carboxy, $C_1$-$C_7$alkoxycarbonyl, phenyl-$C_1$-$C_7$alkoxycarbonyl or $C_1$-$C_7$alkoxy-$C_1$-$C_7$alkoxycarbonyl, carbamoyl which is optionally substituted by a substituent selected from $C_1$-$C_7$alkyl, phenyl-$C_1$-$C_7$alkyl and phenyl; $R_3$ and $R_3'$ represent a common bond; or $R_3$ denotes hydrogen, $C_2$-$C_8$alkanoyl, carboxy-$C_2$-$C_8$alkanoyl, $C_1$-$C_7$alkoxycarbonyl- or$C_1$-$C_7$alkoxy-$C_1$-$C_7$alkoxy-carbonyl-$C_2$-$C_8$alkanoyl, carbamoyl-$C_2$-$C_8$alkanoyl, in which carbamoyl is optionally mono- or disubstituted by a radical selected from $C_1$-$C_7$alkyl and phenyl-$C_1$-$C_7$alkyl, denotes halogeno-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or denotes $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkanesulfonyl or benzene- or naphthylsulfonyl, or denotes $C_1$-$C_7$alkyl or $C_3$-$C_7$alkenyl which are optionally substituted by a radical selected from $C_3$-$C_7$cycloalkyl, phenyl, naphthyl, hydroxyl, $C_1$-$C_7$alkoxy, hydroxy-$C_1$-$C_7$alkoxy, $C_1$-$C_7$alkoxy-$C_1$-$C_7$alkoxy, phenyl-$C_1$-$C_7$alkoxy, phenyl-$C_1$-$C_7$alkoxy-$C_1$-$C_7$alkoxy, amino which is optionally substituted by a substituent selected from $C_1$-$C_7$alkyl, phenyl-$C_1$-$C_7$alkyl and phenyl, carboxy, $C_1$-$C_7$alkoxycarbonyl, phenyl-$C_1$-$C_7$alkoxycarbonyl or $C_1$-$C_7$alkoxy-$C_1$-$C_7$alkoxycarbonyl, carbamoyl which is optionally substituted by a substituent selected from $C_1$-$C_7$alkyl, phenyl-$C_1$-$C_7$alkyl and phenyl; and $R_3'$ denotes hydrogen, $C_1$-$C_7$alkyl or $C_3$-$C_7$alkenyl which are optionally substituted by a radical selected from $C_3$-$C_7$cycloalkyl, phenyl, naphthyl, hydroxyl, $C_1$-$C_7$alkoxy, hydroxy-$C_1$-$C_7$alkoxy, $C_1$-$C_7$alkoxy-$C_1$-$C_7$alkoxy, phenyl-$C_1$-$C_7$alkoxy, phenyl-$C_1$-$C_7$alkoxy-$C_1$-$C_7$alkoxy, amino which is optionally substituted by a substituent selected from $C_1$-$C_7$alkyl, phenyl-$C_1$-$C_7$alkyl and phenyl, carboxy, $C_1$-$C_7$alkoxycarbonyl, phenyl-$C_1$-$C_7$alkoxycarbonyl or $C_1$-$C_7$alkoxy-$C_1$-$C_7$alkoxycarbonyl, carbamoyl which is optionally substituted by a substituent selected from $C_1$-$C_7$alkyl, phenyl-$C_1$-$C_7$alkyl and phenyl; $R_4$ denotes bicycloheptyl, bicycloheptenyl or adamantyl each of which may be substituted by $C_1$-$C_7$alkyl; $R_6$ denotes hydrogen or acetyl; $R_7$ denotes hydrogen or $C_1$-$C_7$alkyl; where the (hetero-)aromatic radicals in each case are unsubstituted or substituted one or more times by a substituent selected from the group consisting of halogen, $C_1$-$C_7$alkyl, $C_1$-$C_7$alkoxy, hydroxyl,

$C_2$-$C_8$alkanoyloxy, trifluoromethyl and nitro.

The invention relates in particular to compounds of the formula I and Ia and their salts, in which the structural elements -$A_1$-$A_2$-, -$A_3$-$A_4$- and -$A_5$-$A_6$- have the meanings given; X represents

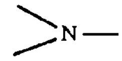

or

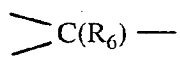

and $R_6$ denotes hydrogen or $C_1$-$C_4$alkyl; A denotes a bond, $C_1$-$C_4$alkylene or, if X represents

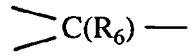 ,

represents the structural element -$X_1$-$A_1$- in which $X_1$ represents -O-, -S-, or -NH- and $A_1$ denotes $C_1$-$C_4$alkylene; ; $R_1$ denotes hydrogen, $C_3$-$C_6$alkanoyl or aminocarbonyl which is optionally mono- or disubstituted by $C_1$-$C_4$alkyl; $R_2$ denotes $C_2$-$C_6$alkanoyl, carboxy-$C_2$-$C_6$alkanoyl, $C_1$-$C_4$alkoxycarbonyl- or $C_1$-$C_4$alkoxy-$C_1$-$C_4$alkoxy-carbonyl-$C_2$-$C_6$alkanoyl, carbamoyl-$C_2$-$C_6$alkanoyl, in which carbamoyl is optionally mono- or disubstituted by a radical selected from $C_1$-$C_4$alkyl and phenyl-$C_1$-$C_4$alkyl, denotes halogeno-$C_2$-$C_6$alkanoyl, $C_1$-$C_4$alkanesulfonyl, or halogeno-$C_1$-$C_4$alkanesulfonyl, or denotes $C_1$-$C_4$alkyl which is optionally substituted by a radical selected from $C_3$-$C_7$cycloalkyl, phenyl, hydroxyl, $C_1$-$C_4$alkoxy, hydroxy-$C_1$-$C_4$alkoxy, $C_1$-$C_4$alkoxy-$C_1$-$C_4$alkoxy, phenyl-$C_1$-$C_4$alkoxy, phenyl-$C_1$-$C_4$alkoxy-$C_1$-$C_4$alkoxy, amino which is optionally substituted by a substituent selected from $C_1$-$C_4$alkyl, phenyl-$C_1$-$C_4$alkyl and phenyl, carboxy, $C_1$-$C_4$alkoxycarbonyl, phenyl-$C_1$-$C_4$alkoxycarbonyl or $C_1$-$C_4$alkoxy-$C_1$-$C_4$alkoxycarbonyl, carbamoyl which is optionally substituted by a substituent selected from $C_1$-$C_4$alkyl, phenyl-$C_1$-$C_4$alkyl and phenyl, or denotes $C_3$-$C_5$akenyl; $R_3$ and $R_3'$ represent a common bond; or $R_3$ denotes hydrogen, $C_2$-$C_6$alkanoyl, carboxy-$C_2$-$C_6$alkanoyl, $C_1$-$C_4$alkoxycarbonyl- or $C_1$-$C_4$alkoxy-$C_1$-$C_4$alkoxy-carbonyl-$C_2$-$C_6$alkanoyl, carbamoyl-$C_2$-$C_6$alkanoyl, in which carbamoyl is optionally mono- or disubstituted by a radical selected from $C_1$-$C_4$alkyl and phenyl-$C_1$-$C_4$alkyl, denotes halogeno-$C_2$-$C_6$alkanoyl,$C_1$-$C_4$alkanesulfonyl, or halogeno-$C_1$-$C_4$alkanesulfonyl, or denotes $C_1$-$C_4$alkyl which is optionally substituted by a radical selected from $C_3$-$C_7$cycloalkyl, phenyl, hydroxyl, $C_1$-$C_4$alkoxy, hydroxy-$C_1$-$C_4$alkoxy, $C_1$-$C_4$alkoxy-$C_1$-$C_4$alkoxy, phenyl-$C_1$-$C_4$alkoxy, phenyl-$C_1$-$C_4$alkoxy-$C_1$-$C_4$alkoxy, amino which is optionally substituted by a substituent selected from $C_1$-$C_4$alkyl, phenyl-$C_1$-$C_4$alkyl and phenyl, carboxy, $C_1$-$C_4$alkoxycarbonyl, phenyl-$C_1$-$C_4$alkoxycarbonyl or $C_1$-$C_4$alkoxy-$C_1$-$C_4$alkoxycarbonyl, carbamoyl which is optionally substituted by a substituent selected from $C_1$-$C_4$alkyl, phenyl-$C_1$-$C_4$alkyl and phenyl, or denotes $C_3$-$C_5$alkenyl; and $R_3'$ denotes hydrogen, $C_1$-$C_4$alkyl or $C_3$-$C_5$alkenyl; $R_4$ denotes bicycloheptyl, bicycloheptenyl or adamantyl each of which may be substituted by $C_1$-$C_4$alkyl; $R_5$ denotes hydrogen or acetyl; $R_7$ denotes hydrogen or $C_1$-$C_4$alkyl; where the aromatic radicals in each case are unsubstituted or substituted one or more times by a substituent selected from the group consisting of halogen, $C_1$-$C_4$alkyl, $C_1$-$C_4$alkoxy, hydroxyl, $C_2$-$C_6$alkanoyloxy, trifluoromethyl and nitro.

The invention relates in particular to compounds of the formula I and Ia and their salts, in which the structural elements -$A_1$-$A_2$-, -$A_3$-$A_4$- and -$A_5$-$A_6$- have the meanings given; X represents

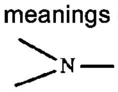

or

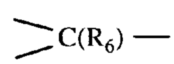

and $R_6$denotes hydrogen or $C_1$-$C_4$alkyl; A denotes $C_1$-$C_4$alkylene, in particular, methylene; $R_1$ denotes hydrogen, $C_2$-$C_6$alkanoyl, in particular, pivaloyl; $R_2$ denotes $C_2$-$C_6$alkanoyl, carboxy-$C_2$-$C_6$alkanoyl, $C_1$-$C_4$alkoxycarbonyl- or $C_1$-$C_4$alkoxy-$C_1$-$C_4$alkoxy-carbonyl-$C_2$-$C_6$alkanoyl, or denotes $C_1$-$C_4$alkyl which is optionally substituted by a radical selected from $C_3$-$C_7$cycloalkyl, phenyl, hydroxyl, $C_1$-$C_4$alkoxy, hydroxy-$C_1$-$C_4$alkoxy, $C_1$-$C_4$alkoxy-$C_1$-$C_4$alkoxy, phenyl-$C_1$-$C_4$alkoxy, phenyl-$C_1$-$C_4$alkoxy-$C_1$-$C_4$alkoxy, carboxy, $C_1$-$C_4$alkoxycarbonyl, phenyl-$C_1$-$C_4$alkoxycarbonyl and $C_1$-$C_4$alkoxy-$C_1$-$C_4$alkoxycarbonyl; $R_3$ and $R_3'$ represent a common bond; or,

in particular, $R_3$ denotes hydrogen, $C_2$-$C_6$alkanoyl, carboxy-$C_2$-$C_6$alkanoyl, $C_1$-$C_4$alkoxycarbonyl- or $C_1C_4$alkoxy-$C_1$-$C_4$alkoxy-carbonyl-$C_2$-$C_6$alkanoyl, or denotes $C_1$-$C_4$alkyl which is optionally substituted by a radical selected from $C_3$-$C_7$cycloalkyl, phenyl, $C_1$-$C_4$alkoxy, hydroxy-$C_1$-$C_4$alkoxy, $C_1$-$C_4$alkoxy-$C_1$-$C_4$alkoxy,phenyl-$C_1$-$C_4$alkoxy, phenyl-$C_1$-$C_4$alkoxy-$C_1$-$C_4$alkoxy, carboxy, $C_1$-$C_4$alkoxycarbonyl, phenyl-$C_1$-$C_4$alkoxycarbonyl and $C_1$-$C_4$alkoxy-$C_1$-$C_4$alkoxycarbonyl; and $R_3'$ denotes hydrogen, $C_1$-$C_4$alkyl or $C_3$-$C_5$alkenyl ; $R_4$ denotes bicycloheptyl, bicycloheptenyl or, in particular, adamantyl each of which may be substituted by $C_1$-$C_4$alkyl; $R_5$ denotes hydrogen or acetyl; $R_7$ denotes hydrogen or $C_1$-$C_4$alkyl;where the aromatic radicals in each case are unsubstituted or substituted one or more times by a substituent selected from the group consisting of halogen, $C_1$-$C_4$alkyl, $C_1$-$C_4$alkoxy, hydroxyl, $C_2$-$C_6$alkanoyloxy, trifluoromethyl and nitro.

The invention relates in particular to compounds of the formula I and Ia and their salts, in which the structural elements -$A_1$-$A_2$-, -$A_3$-$A_4$- and -$A_5$-$A_6$- have the meanings given; X represents

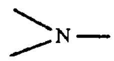

or

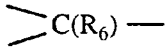

and $R_5$ denotes hydrogen; A denotes $C_1$-$C_4$alkylene, in particular, methylene; $R_1$ denotes hydrogen or branched $C_3$-$C_6$alkanoyl, in particular, pivaloyl; $R_2$ denotes $C_2$-$C_6$alkanoyl, or denotes $C_1$-$C_4$alkyl which is optionally substituted by a radical selected from $C_3$-$C_7$cycloalkyl, phenyl, hydroxyl, $C_1$-$C_4$alkoxy, hydroxy-$C_1$-$C_4$alkoxy, $C_1$-$C_4$alkoxy-$C_1$-$C_4$alkoxy, phenyl-$C_1$-$C_4$alkoxy, phenyl-$C_1$-$C_4$alkoxy-$C_1$-$C_4$alkoxy, carboxy, $C_1$-$C_4$alkoxycarbonyl, phenyl-$C_1$-$C_4$alkoxycarbonyl, and $C_1$-$C_4$alkoxy-$C_1$-$C_4$alkoxycarbonyl; $R_3$ denotes hydrogen, $C_2$-$C_6$alkanoyl, carboxy-$C_2$-$C_6$alkanoyl, $C_1$-$C_4$alkoxycarbonyl- or $C_1$-$C_4$alkoxy-$C_1$-$C_4$alkoxy-carbonyl-$C_2$-$C_6$alkanoyl, or denotes $C_1$-$C_4$alkyl which is optionally substituted by a radical selected from $C_1$-$C_4$alkoxy, $C_1$-$C_4$alkoxy-$C_1$-$C_4$alkoxy, phenyl-$C_1$-$C_4$alkoxy, carboxy, and $C_1$-$C_4$alkoxycarbonyl; and $R_3'$ denotes hydrogen or $C_1$-$C_4$alkyl; $R_4$ denotes bicycloheptyl, bicycloheptenyl or, in particular, adamantyl each of which may be substituted by $C_1$-$C_4$alkyl; $R_5$ denotes hydrogen or acetyl; $R_7$ denotes hydrogen or $C_1$-$C_4$alkyl.

The invention relates in particular to compounds of the formula I and Ia and their salts, in which the structural elements -$A_1$-$A_2$-, -$A_3$-$A_4$- and -$A_5$-$A_6$- have the meanings given; X represents

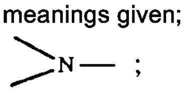

A denotes $C_1$-$C_4$alkylene, in particular, methylene; $R_1$ denotes hydrogen or branched $C_3$-$C_6$alkanoyl, in particular, pivaloyl; $R_2$ denotes $C_2$-$C_6$alkanoyl, such as acetyl, or denotes $C_1$-$C_4$alkyl, such as methyl, which is optionally substituted by a radical selected from $C_1$-$C_4$alkoxy, such as methoxy, $C_1$-$C_4$alkoxy-$C_1$-$C_4$alkoxy, such as (m)ethoxyethoxy, carboxy, $C_1$-$C_4$alkoxycarbonyl, such as (m)ethoxycarbonyl, phenyl-$C_1$-$C_4$alkoxycarbonyl, such benzyloxycarbonyl, and $C_1$-$C_4$alkoxy-$C_1$-$C_4$alkoxycarbonyl, such as (m)ethoxyethoxycarbonyl; $R_3$ denotes hydrogen, $C_2$-$C_6$alkanoyl, such as acetyl, carboxy-$C_2$-$C_6$alkanoyl, such as 3-carboxypropionyl, $C_1$-$C_4$alkoxycarbonyl- or $C_1$-$C_4$alkoxy-$C_1$-$C_4$alkoxy-carbonyl-$C_2$-$C_6$alkanoyl, such as (m)ethoxycarbonylpropionyl or (m)ethoxyethoxycarbonylpropionyl, or denotes $C_1$-$C_4$alkyl, such as methyl, which is optionally substituted by a radical selected from $C_1$-$C_4$alkoxy, such as (m)ethoxy, $C_1$-$C_4$alkoxy-$C_1$-$C_4$alkoxy, such as (m)ethoxyethoxy, carboxy, and $C_1$-$C_4$alkoxycarbonyl, such as (m)ethoxycarbonyl; and $R_3'$ denotes hydrogen or $C_1$-$C_4$alkyl, such as methyl; $R_4$ denotes adamantyl, such as 1-adamantyl; $R_5$ denotes hydrogen or acetyl; $R_7$ denotes hydrogen or $C_1$-$C_4$alkyl, such as methyl.

The invention relates in particular to compounds of the formula I and Ia and their salts, in which the structural elements -$A_1$-$A_2$-, -$A_3$-$A_4$- and -$A_5$-$A_6$- have the meanings given; X represents

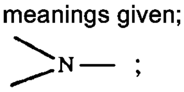

A denotes methylene; $R_1$ denotes pivaloyl; $R_2$ denotes $C_2$-$C_6$alkanoyl, such as acetyl, or denotes $C_1$-$C_4$alkyl, such as methyl, which is optionally substituted by a radical selected from $C_1$-$C_4$alkoxy, such as methoxy, $C_1$-$C_4$alkoxy-$C_1$-$C_4$alkoxy, such as methoxyethoxy; $R_3$ denotes hydrogen, $C_2$-$C_6$alkanoyl, such as acetyl, carboxy-$C_2$-$C_6$alkanoyl, such as 3-carboxypropionyl, or $C_1$-$C_4$alkoxycarbonyl-$C_2$-$C_6$alkanoyl, such as 3-methoxycarbonylpropionyl, or denotes $C_1$-$C_4$alkyl, such as methyl, which is optionally substituted by a radical selected from $C_1$-$C_4$alkoxy, such as methoxy, $C_1$-$C_4$alkoxy-$C_1$-$C_4$alkoxy, such as methoxyethoxy, carboxy, and $C_1$-$C_4$alkoxycarbonyl, such methoxycarbonyl; and $R_3'$ denotes hydrogen, also $C_1$-$C_4$alkyl, such as methyl; $R_4$ denotes adamantyl, such as 1-adamantyl; $R_5$ denotes acetyl; $R_7$ denotes hydrogen or $C_1$-$C_4$alkyl, such as

methyl.

The invention relates in particular to compounds of the formula I and Ia and their salts, in which the structural elements $-A_1-A_2-$, $-A_3-A_4-$ and $-A_5-A_6-$ have the meanings given; X represents

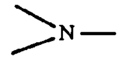 ;

A denotes methylene; $R_1$ denotes amino-carbonyl which is optionally mono- or disubstituted by $C_1$-$C_4$alkyl, such as N,N-dimethylcarbamoyl; $R_2$ denotes $C_2$-$C_6$alkanoyl, such as acetyl, or denotes $C_1$-$C_4$alkyl, such as methyl, which is optionally substituted by a radical selected from $C_1$-$C_4$alkoxy, such as methoxy, $C_1$-$C_4$alkoxy-$C_1$-$C_4$alkoxy, such as methoxyethoxy; $R_3$ denotes hydrogen, $C_2$-$C_6$alkanoyl, such as acetyl, carboxy-$C_2$-$C_6$alkanoyl, such as 3-carboxypropionyl, or $C_1$-$C_4$alkoxycarbonyl-$C_2$-$C_6$alkanoyl, such as 3-methoxycarbonylpropionyl, or denotes $C_1$-$C_4$alkyl, such as methyl, which is optionally substituted by a radical selected from $C_1$-$C_4$alkoxy, such as methoxy, $C_1$-$C_4$alkoxy-$C_1$-$C_4$alkoxy, such as methoxyethoxy, carboxy, and $C_1$-$C_4$alkoxycarbonyl, such methoxycarbonyl; and $R_3'$ denotes hydrogen, also $C_1$-$C_4$alkyl, such as methyl; $R_4$ denotes adamantyl, such as 1-adamantyl; $R_5$ denotes acetyl; $R_7$ denotes hydrogen or $C_1$-$C_4$alkyl, such as methyl.

The invention relates in particular to compounds of the formula I and Ia and their salts, in which the structural elements $-A_1-A_2-$, $-A_3-A_4-$ and $-A_5-A_6-$ have the meanings given; X represents

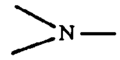 ;

A denotes methylene; $R_1$ denotes pivaloyl; $R_2$ denotes $C_1$-$C_4$alkyl, such as methyl, which is optionally substituted by a radical selected from $C_1$-$C_4$alkoxy, such as methoxy, and $C_1$-$C_4$alkoxy-$C_1$-$C_4$alkoxy, such as methoxyethoxy; $R_3$ and $R_3'$ denote hydrogen; $R_4$ denotes adamantyl, such as 1-adamantyl; $R_5$ denotes acetyl; $R_7$ denotes hydrogen.

The invention relates in particular to compounds of the formula I and Ia and their salts, in which the structural elements $-A_1-A_2-$, $-A_3-A_4-$ and $-A_5-A_6-$ have the meanings given; X represents

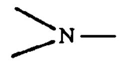 ;

A denotes methylene; $R_1$ denotes pivaloyl; $R_2$ denotes $C_1$-$C_4$alkyl, such as methyl; $R_3$ and $R_3'$ denote hydrogen; $R_4$ denotes adamantyl, such as 1-adamantyl; $R_5$ denotes acetyl; $R_7$ denotes hydrogen.

The invention particularly relates to the novel compounds mentioned in the examples, and the preparation thereof.

The invention likewise relates to processes for the preparation of the compounds according to the invention. The preparation of compounds of the formula I and Ia and salts thereof is carried out in a manner known per se and is characterized in that, for example,

a) for the preparation of compounds of the formula I and Ia and salts thereof, in which X represents

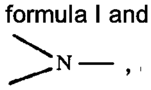 ,

a compound of the formula

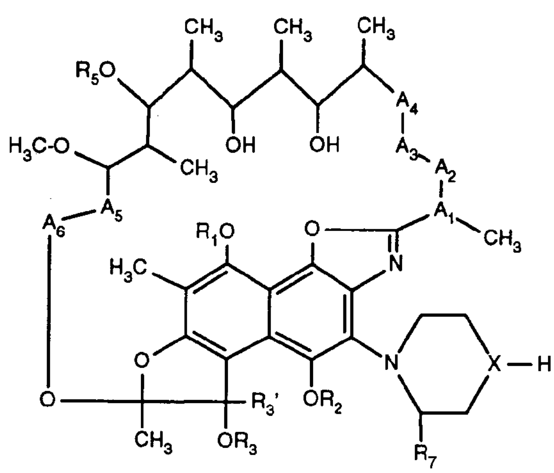

(IIa)

or a salt thereof, in which X represents

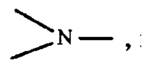

is reacted with a compound of the formula

Z-A-$R_4$ (IIb)

in which Z denotes reactive esterified hydroxyl, or

b) a compound of the formula

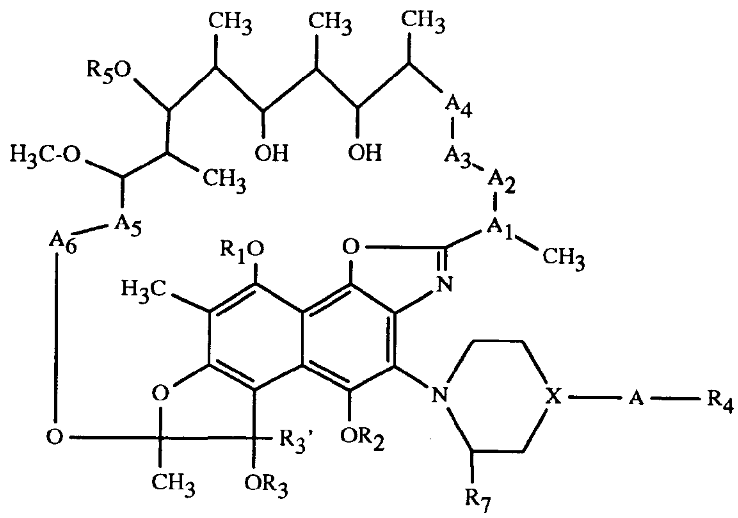

(III)

in which $R_2$ is hydrogen, is acylated or alkylated, or

c) a compound of the formula

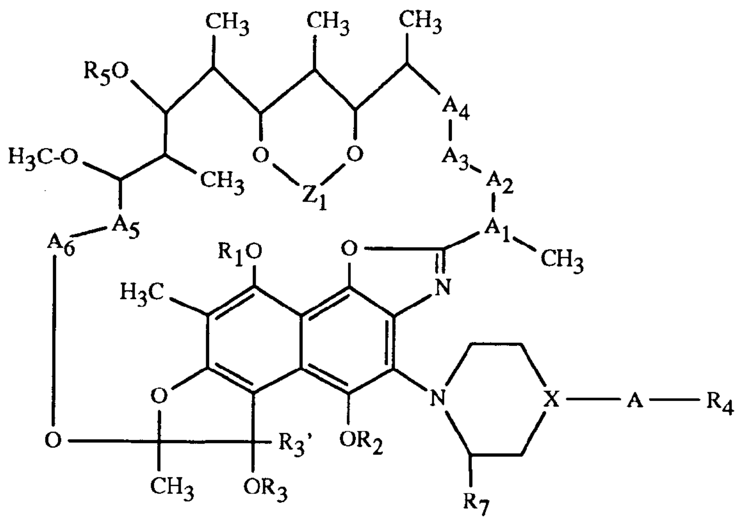

(IV)

in which $Z_1$ denotes alkylidene or cycloalkylidene, is hydrolysed, and, if desired, a compound of the formula I and Ia obtainable according to the process or by other means, or a salt thereof, is converted into another compound according to the invention, or a salt thereof, a free compound of the formula I and Ia obtainable according to the process is converted into a salt and/or a salt obtainable according to the process is convened into the free compound of the formula I and Ia or into another salt and, if desired, a mixture of isomers obtainable according to the process is fractionated.

Salts of the starting materials of the formulae IIa, III and IV which have an acidic phenolic hydroxyl group are appropriate salts with bases of the type detailed hereinbefore, whereas the starting compounds of the formula IIa, III or IV which have one or two basic centres can form corresponding acid addition salts in analogy to the acid addition salts of the formula I.

Reactive esterified hydroxyl, for example Z, is, in particular, hydroxyl which is esterified with a strong inorganic acid or organic sulfonic acid, for example halogen such as chlorine, bromine or iodine, sulfonyloxy such as hydroxysulfonyloxy, halogenosulfonyloxy, for example fluorosulfonyloxy, optionally substituted, for example by halogen, $C_1$-$C_7$alkanesulfonyloxy, for example methane- or trifluoromethanesulfonyloxy, $C_5$-$C_7$cycloalkanesulfonyloxy, for example cyclohexanesulfonyloxy, or optionally substituted, for example by $C_1$-$C_7$alkyl or halogen, benzenesulfonyloxy, for example p-bromobenzene- or p-toluenesulfonyloxy.

Alkylidine denotes, for example, $C_1$-$C_7$alkylidene, in particular $C_1$-$C_5$alkylidene such as methylene, ethylidene, isopropylidene, 1-methyl-propylidene or -butylidene, whereas cycloalkylidene denotes, for example, $C_3$-$C_7$cycloalkylidene, in particular cyclopentylidene or -hexylidene.

The reactions described hereinbefore and hereinafter in the variants are carried out in a manner known per se, for example, in the absence or customary manner in the presence of a suitable solvent or diluent or of a mixture thereof, and they are carried out, as required, with cooling, at room temperature or with heating, for example in a temperature range from about -80° up to the boiling point of the reaction medium, preferably from about - 10° up to about + 180°C and, if necessary, in a closed vessel, under pressure, in an inert gas atmosphere and/or under anhydrous conditions.

Variant a):

Z preferably denotes halogen, such as chlorine, bromine or iodine, furthermore sulfonyloxy such as methane- or p-toluenesulfonyloxy.

The reaction is carried out in a manner known per se, advantageously in the presence of a base.

Suitable and preferred bases are non-nucleophilic tertiary amines, for example tri-lower-alkylamines, basic heterocycles and carbocyclic amines such as ethyl-diisopropylamine, triethylamine, pyridine, 1,5-diaza-bicyclo[4.3.0]non-5-ene (DBN) and 1,8-diaza-bicyclo[5.4.0]undec-7-ene (DBU).

The manufacture of starting material of the formula IIa in which X is protected, can be carried out e.g. using the method described in variant b) in EP 350 445.

Variant b):

The acylation is carried out in a manner known per se using a suitable acylating agent. An example of a suitable acylating agent is a compound of the formula Ac-$Z_2$ (IIIa), where Ac denotes an acyl radical corresponding to variable $R_2$ and $Z_2$ denotes hydroxyl or, in particular, reactive activated hydroxyl. Appropriate hydroxyl can be activated, for example, by strong acids such as hydrohalic or carboxylic acid, for example by hydrochloric, hydrobromic acid, an optionally substituted, for example by halogen, alkanecarboxylic acid or by an acid of the formula Ac-OH, or by suitable activating or coupling reagents of the type detailed hereinafter, in particular in situ. Ac-$Z_2$ can furthermore represent an activated ester, where $Z_2$ denotes, in particular, cyanomethoxy, (4-)nitrophenoxy or polyhalogeno-, such as pentachloro-, -phenoxy. Activating and coupling reagents which can be employed are, in particular, carbodiimides, for example N,N′-di-$C_1$-$C_4$alkyl- or N,N′-di-$C_5$-$C_7$cycloalkyl-carbodiimide, such as diisopropylcarbodiimide or N,N′-dicyclohexylcarbodiimide, advantageously with the addition of an activating catalyst such as N-hydroxysuccinimide or optionally substituted, for example by halogen, $C_1$-$C_7$alkyl or $C_1$-$C_7$alkoxy, N-hydroxy-benzotriazole or N-hydroxy-5-norbornene-2,3-dicarboxamide, furthermore $C_1$-$C_4$alkyl halogenoformate, for example isobutyl chloroformate, suitable carbonyl compounds, for example N,N-carbonyldiimidazole, suitable 1,2-oxazolium compounds, for example 2-ethyl-5-phenyl-1,2-oxazolium 3′-sulfonate or 2-tert-butyl-5-methyl-isoxazolium perchlorate, suitable acylamino compounds, for example 2-ethoxy-1-ethoxycarbonyl- 1,2-dihydroquinoline, or suitable phosphoryl cyanamides or azides, for example diethylphosphoryl cyanamide or diphenylphosphoryl azide, furthermore triphenylphosphine disulfide or 1-$C_1$-$C_4$alkyl-2-halogeno-pyridinium halides, for example 1-methyl-2-chloropyridinium iodide.

$Z_2$ preferably denotes halogen such as chlorine or bromine, and Ac-O-.

The acylation according to the invention is preferably carried out under mild conditions, for example at room temperature or slightly elevated temperatures. Depending on the nature of the starting material of the formula III, the amount of acylating agent must be selected so that one, two or, secondarily, three acyl groups are introduced. The acylating agent can advantageously act as solvent. The course of the reaction is expediently followed by customary analytical methods, in particular using thin-layer chromatography.

If the acylation is carried out with a compound of the formula Ac-$Z_2$ in which $Z_2$ denotes halogen, it is advantageously carried out in the presence of an acid-binding agent such as a base which cannot be acylated. Suitable bases are, for example, alkali metal hydroxides, hydrides, amides, alkanolates, carbonates,

triphenylmethylides, di(lower alkyl)amides, aminoalkylamides or lower alkyl silylamides, or naphthaleneamines, lower alkylamines, basic heterocycles, ammonium hydroxides, and also carbocyclic amines. Examples which may be mentioned are sodium hydroxide, sodium hydride, sodium amide, sodium (m-)ethoxide, potassium tert-butoxide, caesium carbonate, potassium carbonate, lithium triphenylmethylide, lithium diisopropylamide, potassium 3-(aminopropyl)amide, potassiumbis(trimethylsilyl)amide, dimethylaminonaphthalene, di- or triethylamine, or ethyldiisopropylamine, N-methylpiperidine, pyridine, benzyltrimethylammonium hydroxide, 1,5-diazabicyclo[4.3.0]non-5-ene (DBN) and 1,8-diazabicyclo[5.4.0]undec-7-ene (DBU). In the reaction with an anhydride, in particular a symmetric anhydride, an excess of Ac-O-Ac is used, in particular.

A preferred embodiment of the acylation process according to the invention starts from those compounds of the formula III in which $R_1$ denotes acyl, $R_5$ is acetyl, and $R_2$ and $R_3$ denote hydrogen. Depending on the choice of reaction conditions, the acylation can be controlled in such a way that only the oxygen atom on C-4 of the rifamycin ring system is acylated. This entails, in particular, acylation at room temperature with the particular acylating agent, which simultaneously acts as solvent. If the temperature is raised, or if, for example, a compound of the formula IIIa is employed while using a base in which $Z_2$ denotes halogen, the O atom on C-4 and C-11, in particular, are simultaneously acylated.

If $R_2$ denotes hydrogen, this phenolic 4-hydroxyl group can be etherified in a manner known per se. The etherification can be carried out, for example, with a reactive ester of the aliphatic alkanol, such as an alkanol which is optionally substituted as indicated. Examples of suitable reactive esters of the relevant alcohols are those with strong inorganic or organic acids, such as appropriate halides, sulfates, sulfonates, for example lower alkanesulfonates or optionally substituted benzenesulfonates, in particular chlorides, bromides, iodides, methane-, benzene- or p-toluenesulfonates. The etherification can be carried out, for example, in the presence of a base such as an alkali matel hydride, hydroxide, carbonate or of a basic amine, such as sodium borhydride or potassium carbonate. It is advantageous to start from an appropriate alkali metal salt of the formula III.

If $R_2$ denotes alkanoyl and $R_3$ denotes hydrogen, the corresponding 4-O-acyl derivative may be in equilibrium with the corresponding 11-O-acyl derivative, that is to say the 4-O-acyl group migrates to the 11-hydroxyl group.

If both $R_2$ and $R_3$ denote hydrogen, under usual reaction conditions, the 4-OH group is preferably esterified or etherified, respectively, first.

The preparation of the starting material of the formula III starts, for example, from rifamycin S or SV and introduces the side-chain

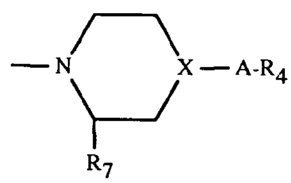

in position 3 in a manner known per se, for example as described in WO 87/02361. The appropriate SV derivative is subsequently converted using an acylating agent, such as pivaloyl chloride, into the corresponding 1,8-di-O-acyl compound and then, by prolonged heating, for example at 100°C, into the corresponding 8-O-acyl-1-deoxy-15-deoxo-1, 15-oxy derivative of the formula III, for example, analogously to the corresponding methods as described in EP 350 445. Corresponding hydro compounds can be prepared by hydrogenation in a manner known per se, for example as described hereinafter.

Variant c):

$Z_1$ primarily represents $C_1$-$C_5$alkylidene such as isopropylidene. The ketal of the formula IV is hydrolysed in a manner known per se, advantageously using an acid such as an inorganic or organic acid, such as mineral acid, for example a hydrohalic acid, sulfuric acid or a phosphorus acid, sulfonic acid, for example methane- or p-toluenesulfonic acid, or a carboxylic acid, for example acetic acid.

The preparation of compounds of the formula IV is carried out in a manner known per se. Thus, for example, it starts from a compound of the formula I and Ia in which $R_2$ is hydrogen and $R_3$ and $R_3'$ together represent a bond or each denote hydrogen, and the latter is reacted in the presence of one of the listed acids with an aldehyde or ketone, or a ketal thereof, such as di-$C_1$-$C_4$alkyl or $C_2$-$C_4$alkylene ketal, for example acetone dimethyl ketal, corresponding to $Z_1$. In turn, compounds of the formula I and Ia in which $-A_1$-$A_2$-, $-A_3$-$A_4$- and $-A_5$-$A_6$- each denote vinylene, and $R_3$ and $R_3'$ together represent a bond, are described, for example, in EP

314624. Corresponding hydro derivatives (-$A_1$-$A_2$-, -$A_3$-$A_4$- and -$A_5$-$A_6$- denote ethylene, for example) can be obtained, for example, using the hydrogenation processes detailed hereinafter.

The invention likewise relates to the novel compounds obtainable by the abovementioned process variants.

A compound of the formula I and Ia obtainable according to the invention or in another manner, or salt thereof, can be converted in a manner known per se into another compound of the formula I and Ia.

Compounds of the formula I and Ia in which the structural elements -$A_1$-$A_2$-, -$A_3$-$A_4$- and -$A_6$-$A_6$- each denote vinylene or -$A_1$-$A_2$- and -$A_3$-$A_4$- each denote ethylene and -$A_6$-$A_6$-denotes vinylene can be converted by reduction, for example by catalytic hydrogenation, into the corresponding tetrahydro-(-$A_1$-$A_2$- and -$A_3$-$A_4$- are each ethylene and -$A_6$-$A_6$- is vinylene) or the corresponding hexahydro derivatives (-$A_1$-$A_2$-, -$A_3$-$A_4$- and -$A_5$-$A_6$- each denote ethylene). Thus the hydrogenation of the multiple bonds takes place in the presence of hydrogenation catalysts, suitable for this purpose being, for example, noble metals or derivatives thereof, for examples oxides, such as nickel, Raney nickel, palladium, platinum oxide, which can optionally be absorbed on support materials, for example on charcoal or calcium carbonate. The hydrogenation can be carried out, in particular, under pressures of 1 to about 100 at.

The 11-oxo group in compounds of the formula I and Ia, in which $R_3$ and $R_3'$ represent a common bond, can be reduced in manner known per se, for example, by catalytic hydrogenation in the presence of hydrogenation catalysts, e.g. as mentioned above, or using suitable hydrides, such as alkali metal boro hdrides, such as sodium borohydride.

Compounds of the formula I and Ia, in which $R_3$ and $R_3'$ represent a common bond, can be converted to compounds of the formula I and Ia, in which $R_3$ denotes hydrogen and $R_3'$ denotes an aliphatic radical, for example, by using conventional alkylation methods, such as Grignard reaction, for example, using an organomagnesium-halide or an organo-lithium compound, or, if starting from compounds of the formula I and Ia, in which $R_3'$ denotes hydrogen, by reaction with a corresponding reactive derivative of an aliphatic alcohol, e.g. a halide thereof, in the presence of suitable base.

Compounds of the formula I and Ia in which, for example one of the radicals $R_1$ and $R_3$ denotes hydrogen, and at least one of them can be acylated in a manner known per se, for example in analogy to the methods described in variant b), for example by reaction with the appropriate carboxylic acid or with a reactive derivative thereof. Examples of reactive derivatives of this type are anhydrides, including mixed anhydrides, such as an acid halide, for example chloride, or anhydrides with a formic ester, activated carboxylic esters such as cyanomethyl, (4-)nitrophenyl, polyhalogenophenyl, for example pentachlorophenyl, esters. The reaction with the carboxylic acid or a salt thereof takes place under water-eliminating conditions, for example with azeotropic removal of the water of reaction, or by treatment with a suitable condensing agent, for example N,N′-dicyclohexyl-carbodiimide. The reaction with a reactive acid derivative is advantageously carried out in the presence of a base. Correspondingly, the acetyl radical $R_5$ can be introduced into compounds of the formula I and Ia in which $R_5$ is hydrogen by treatment with an appropriate acetylating agent.

It is possible by treatment with strong bases, such as alkali metal hydroxides, to replace the acetyl radical $R_5$ and the acyl radical $R_1$, $R_2$ and/or $R_3$ by hydrogen. The acyl radical $R_1$ can also be selectively eliminated in the presence of the acetyl radical $R_5$, for example by treatment with a fluoride such as alkali metal, for example sodium or cesium fluoride, or with an ammonium fluoride, for example tetrabutylammonium fluoride.

Acetyl $R_5$ can selectively be replaced by hydrogen in the presence of other acyl groups, for example, if $R_1$ denotes pivaloyl, by treatment with hydrazine (hydrate) or a suitable derivative thereof.

Compounds of the formula I and Ia, in which e.g. $R_2$ and/or $R_3$ denote an aliphatic radical, can be converted in a manner known per se into those compounds of the formula I and Ia in which $R_2$ and/or $R_3$ are hydrogen. The ether cleavage can be carried out, for example, using strong acids such as mineral acids, for example the hydrohalic acids hydrobromic or hydroiodic acid, which can advantageously be in the form of pyridinium halides, or using Lewis acids, for example halides of elements of the 3rd main group or corresponding subgroups of the periodic table of elements. If, for example, $R_2$ and/or $R_3$ denote benzyl, the latter can be eliminated using methods known per se, for example by catalytic hydrogenation, for example in the manner described hereinbefore. These reactions can, if necessary, be carried out while cooling or heating, for example in a temperature range from about -20° to about 100°C, in the presence or absence of a solvent or diluent, under inert gas and/or under pressure and, where appropriate, in a closed vessel.

A compound according to the invention containing hydroxyl can be etherified by methods known per se, e.g. as indicated above. The etherification can be carried out, for example, using an alcohol, such as a substituted or unsubstituted lower alkanol, or a reactive ester thereof. Possible reactive esters of the desired alcohols are, for example, those with strong inorganic or organic acids, such as corresponding halides, sulfates, lower alkanesulfonates or substituted or unsubstituted benzenesulfonates, for example chlorides, bromides, iodides, methane-, benzene- or p-toluenesulfonates. The etherification can be carried out, for example, in the presence of a base, an alkali metal hydride, hydroxide or carbonate, or a basic amine. Inversely, corresponding ethers,

such as lower alkoxy compounds, can be cleaved, for example, by means of strong acids, such as mineral acids, for example the hydrohalic acids, hydrobromic or hydriodic acid, which may advantageously be present in the form of pyridinium halides, or by means of Lewis acids, for example halides of elements of main group III or the corresponding sub-groups. These reactions can be carried out, if necessary, with cooling or warming, for example in a temperature range of about -20° to about 100°C, in the presence or absence of a solvent or diluent, under inert gas and/or under pressure and, if appropriate, in a closed vessel.

If one of the variables (for example $R_2$ and $R_3$) contains amino, corresponding compounds of the formula I and Ia, their tautomers or salts can be N-alkylated in a manner known per se; likewise, carbamoyl or radicals (for example $R_2$) containing carbamoyl can be N-alkylated. The (aryl)alkylation is carried out, for example, using a reactive ester of an (aryl)$C_1$-$C_7$alkyl halide, for example a bromide or iodide, an (aryl)$C_1$-$C_7$alkylsulfonate, for example a methanesulfonate or p-toluenesulfonate, or a di-$C_1$-$C_7$alkyl sulfate, for example dimethyl sulfate, preferably under basic conditions, such as in the presence of sodium hydroxide solution or potassium hydroxide solution, and advantageously in the presence of a phase-transfer catalyst, such as tetrabutylammonium bromide or benzyltrimethylammonium chloride, where, however, stronger basic condensing agents, such as alkali metal amides, hydrides or alkoxides, for example sodium amide, sodium hydride or sodium ethoxide, may be necessary.

In compounds of the formula (I) which contain an esterified or amidated carboxyl group (for example $R_2$ or $R_3$) as a substituent, a group of this type can be converted into a free carboxyl group, for example by means of hydrolysis, for example in the presence of a basic agent, or an acidic agent, such as a mineral acid. Tert-butyloxycarbonyl, for example, may furthermore be converted into carboxyl, for example in a manner known per se, such as by treating with trihaloacetic acid, such as trifluoroacetic acid, and benzyloxycarbonyl may be converted into carboxyl, for example by catalytic hydrogenation in the presence of a hydrogenation catalyst, for example in the manner described below.

Furthermore, in compounds of the formula (I) which contain a carboxyl group (for example $R_3$) as a substituent, this can be converted into an esterified carboxyl group (for example $R_3$), for example, by treating with an alcohol, such as a lower alkanol, in the presence of a suitable esterifying agent, such as an acid reagent, for example an inorganic or organic acid or a Lewis acid, for example zinc chloride, or a condensing agent which combines with water, for example a carbodiimide, such as N,N'-dicyclohexylcarbodiimide, or by treating with a diazo reagent, such as with a diazo-lower alkane, for example diazomethane. This can also be obtained if compounds of the formula I and Ia in which the carboxyl group (for example $R_2$) is present in free form or in salt form, such as ammonium salt or metal salt, for example alkali metal salt, such as sodium salt or potassium salt form, are treated with a reactive ester of a ($C_1$-$C_7$)alkyl halide, for example methyl or ethyl bromide or iodide, or an organic sulfonic acid ester, such as an appropriate ($C_1$-$C_7$)alkyl ester, for example methyl or ethyl methanesulfonate or p-toluenesulfonate.

Compounds of the formula (I) which contain an esterified carboxyl group (for example $R_3$) as a substituent can be transesterified into other ester compounds of the formula (I) by transesterification, for example by treating with an alcohol, customarily a higher appropriate alcohol than that of the esterified carboxyl group in the starting material, in the presence of a suitable transesterifying agent, such as a basic agent, for example an alkali metal ($C_1$-$C_7$)alkanoate, ($C_1$-$C_7$)alkanolate or cyanide, such as sodium acetate, sodium methoxide, sodium ethoxide, sodium tert-butoxide or sodium cyanide, or a suitable acid agent, if appropriate with removal of the resulting alcohol, for example by distillation. Appropriate, so-called activated esters of the formula (I) may also be used which contain an activated esterified carboxyl group as a substituent (see below), and these may be converted into another ester by treating with a ($C_1$-$C_7$)alkanol.

In compounds of the formula (I) which contain the carboxyl group (for example $R_2$) as a substituent, this can also first be converted into a reactive derivative, such as an anhydride, including a mixed anhydride, such as an acid halide, for example an acid chloride (for example by treating with a thionyl halide, for example thionyl chloride), or an anhydride using a formic acid ester, for example a ($C_1$-$C_7$)alkyl ester (for example by treating a salt, such as an ammonium or alkali metal salt, with a haloformic acid ester, such as a chloroformic acid ester, such as a ($C_1$-$C_7$)alkyl ester), or into an activated ester, such as a cyanomethyl ester, a nitrophenyl ester, for example a 4-nitrophenyl ester, or a polyhalophenyl ester, for example a pentachlorophenyl ester (for example by treating with an appropriate hydroxyl compound in the presence of a suitable condensing agent, such as N,N'-dicyclohexylcarbodiimide), and then a reactive derivative of this type can be reacted with an amine and in this way amide compounds of the formula (I) which contain an amidated carboxyl group as a substituent can be obtained. In this case, these can be obtained directly or via intermediate compounds; thus, for example, an activated ester, such as a 4-nitrophenyl ester or a compound of the formula I and Ia containing a carboxyl group can first be reacted with a 1 -unsubstituted imidazole and the 1-imidazolylcarbonyl compound obtained in this way brought to reaction with an amine. However, other non-activated esters, such as ($C_1$-$C_7$)alkyl esters of compounds of the formula (I), which contain, for example, ($C_2$-$C_8$)alkoxycarbonyl (for example $R_2$) as a substituent,

can also be brought to reaction with amines.

Salts of compounds of the formula (I) can be prepared in a manner known per se. Thus, for example, acid addition salts of compounds of the formula (I) are obtained by treatment with an acid or with a suitable ion exchanger reagent. Salts can be converted in a customary manner into the free compounds, acid addition salts, for example, by treatment with a suitable basic agent.

Depending on the procedure and reaction conditions, the compounds according to the invention with salt-forming, in particular basic, properties can be obtained in free form or, preferably, in the form of salts.

As a consequence of the close relationship between the novel compound in free form and in the form of salts thereof, hereinbefore and hereinafter by the free compound or salts thereof is meant, where appropriate for the sense and purpose, also the correspondingsalts or the free compound.

The novel compounds, including their salts of salt-forming compounds, can also be obtained in the form of hydrates thereof, or include other solvents used for crystalization.

The novel compounds can, depending on the choice of starting materials and procedures, be in the form of one of the possible isomers or as mixtures thereof, for example depending on the number of asymmetric carbon atoms, as pure optical isomers, such as antipodes, or as mixtures of isomers, such as racemates, diastereoisomeric mixtures or racemate mixtures.

Racemate mixtures which are obtained can be separately fractionated on the basis of the physicochemical differences of the components in a known manner into the pure isomers or racemates, for example by fractional crystallization. Racemates which are obtained can, furthermore, be resolved by known methods into the optical antipodes, for example by recrystallization from an optically active solvent, chromatography on chiral adsorbents, using suitable microorganisms, by cleavage with specific, immobilized enzymes, via the formation of inclusion compounds, for example using chiral crown ethers, in which case only one enantiomer is complexed, or by conversion into diastereomeric salts, for example by reaction of a basic final compound racemate with an optically active acid such as carboxylic acid, for example tartaric or mallic acid, or sulfonic acid, for example camphorsulfonic acid, and separation of the diastereomeric mixture obtained in this manner, for example on the basis of their different solubilities, into the diastereomers, from which the desired enantiomer can be liberated by the action of suitable agents. The more active enantiomer is advantageously isolated.

The wording up of the reaction product from the reaction mixture obtainable according to the process is carried out in a manner known per se, for example by dilution with water, and/or where appropriate by neutralization or slight acidification (to about pH = 3) with an aqueous acid, such as an inorganic or organic acid, for example a mineral acid or, advantageously, citric acid, and addition of a solvent which is immiscible with water, such as chlorinated hydrocarbon, for example chloroform or methylene chloride, in which case the reaction product transfers into the organic phase, from which it can be obtained in pure form in a customary manner, for example by drying, evaporation of the solvent and crystallization and/or chromatography of the residue or other customary purification methods. If the above reaction yields, for example, a mixture of acylated compounds, the latter can be fractionated in a manner known per se, for example by fractional crystallization, chromatography etc., into the desired individual acyl compounds.

The invention also relates to those embodiments of the process which start from a compound obtainable at any one stage of the process as intermediate, and the missing steps are carried out or a starting material is used in the form of a derivative or salt and/or its racemates or antipodes, or, in particular, is formed under the reaction conditions.

In the process of the present invention, the starting materials which are preferably used are those which lead to the compounds described as particularly valuable in the introduction. The invention likewise relates to novel starting materials which have been developed specifically for the preparation of the compounds according to the invention, in particular novel compounds of the formula III, the use thereof and processes for the preparation thereof, where the variables $-A_1-A_2-$, $-A_3-A_4-$, $-A_5-A_6-$, $R_1$, $R_2$, $R_3$, $R_3'$, $R_4$, $R_5$, $R_6$, $R_7$, X and A have the meanings indicated for the preferred groups of compounds of the formula I and Ia in each case. Especially preferred as starting material are compounds of the formula III and salts thereof, in which $R_2$ denotes hydrogen; $R_3$ and $R_3'$ represent a common bond or each are hydrogen, being an additional subject matter of the invention;

The present invention also embraces the use of compounds of the formula I and Ia and salts thereof alone or together with auxiliaries, as well as in combination with other active compounds, as agents for the therapeutic treatment, namely both curative and preventive, of diseases or pathological states indicated or caused, for example, by an elevated content of cholesterol and/or triglycerides in the blood, in particular in blood serum. The active compounds according to the invention are administered in therapeutically effective amounts, preferably in the form of pharmaceutical compositions together with conventional pharmaceutical vehicles and/or auxiliaries, to the warm-blooded animals, primarily humans, requiring treatment. This entails, for example, administration to warm-blooded animals, depending on the species, body weight, age and individual condition, of daily doses corresponding to about 1 to about 100, in particular about 3 to about 50, mg per kg of body weight,

which can be exceeded in severe cases. Accordingly, the invention also embraces the corresponding method for medical treatment.

The invention likewise relates to pharmaceutical products which contain the compounds according to the invention, or pharmaceutically utilizable salts thereof, as active compounds, and to processes for the preparation thereof.

The pharmaceutical products according to the invention which contain the compound according to the invention or pharmaceutically utilizable salts thereof are those for enteral, such as oral furthermore rectal, and parenteral administration to warm-blooded animal(s), the pharmacological active compound being contained alone or together with a pharmaceutically utilizable vehicle. The daily dosage of the active compound depends on the age and the individual condition as well as on the mode of administration.

The novel pharmaceutical products contain, for example, from about 10 % to about 80 %, preferably from about 20 % to about 60 %, of the active compound. Examples of pharmaceutical products according to the invention for enteral or parenteral administration are those in dose-unit forms such as coated tablets, tablets, capsules or suppositories, as well as ampoules. These are prepared in a manner known per se, for example using conventional mixing, granulating, coating, dissolving or freeze-drying processes. Thus, pharmaceutical products for oral use can be obtained by combining the active compound with solid excipients, where appropriate granulating a mixture which is obtained, and processing the mixture or granules, if desired or necessary, after addition of suitable auxiliaries to tablets or cores of coated tablets.

Particularly suitable excipients are fillers such as sugars, for example lactose, sucrose, mannitol or sorbitol, cellulose products and/or calcium phosphates, for example tricalcium phosphate or calcium hydrogen phosphate, as well as binders such as starch mucillage using, for example, maize, wheat, rice or potato starch, gelatin, tragacanth, methylcellulose and/or polyvinylpyrrolidone, if desired, disintegrants such as the abovementioned starches, as well as carboxymethyl starch, crosslinked polyvinylpyrrolidone, agar, alginic acid or a salt thereof, such as sodium alginate, auxiliaries are primarily flowability and flow-regulating agents and lubricants, for example silica, talc, stearic acid or salts thereof, such as magnesium or calcium stearate, and/or polyethylene glycol. Cores of coated tablets are provided with suitable, optionally enteric, coatings, using, inter alia, concentrated sugar solutions which optionally contain gum arabic, talc, polyvinylpyrrolidone, polyethylene glycol and/or titanium dioxide, lacquer solutions in suitable organic solvents or solvent mixtures or, for the preparation of enteric coatings, solutions of suitable cellulose products such as acetyl cellulose phthalate or hydroxypropylmethylcellulose phthalate. Colorants or pigments can be added to the tablets or coatings of coated tablets, for example, to identify or to indicate various doses of active compound.

Further pharmaceutical products which can be used orally are two-piece capsules made of gelatin, as well as soft, closed capsules made of gelatin and a plasticizer such as glycerol or sorbitol. The two-piece capsules can contain the active compound in the form of granules, for example mixed with fillers such as lactose, binders such as starches, and/or glidants such as talc or magnesium stearate, and, where appropriate, stabilizers. The active compound in soft capsules is preferably dissolved or suspended in suitable liquids such as fatty oils, liquid paraffin or liquid polyethylene glycols, it likewise being possible to add stabilizers.

Examples of pharmaceutical products suitable for rectal use are suppositories which consist of a combination of the active compound and a suppository base. Examples of suitable suppository bases are natural or synthetic triglycerides, paraffin hydrocarbons, polyethylene glycols or higher alkanols. It is furthermore possible to use gelatin rectal capsules which contain a combination of the active compound with a base substance. Examples of suitable base substances are liquid triglycerides, polyethylene glycols or paraffin hydrocarbons.

Primarily suitable for parenteral administration are aqueous solutions of an active compound in water-soluble form, for example of a water-soluble salt, furthermore suspensions of the active compound such as appropriate oily injection suspensions, in which case suitable lipophilic solvents or vehicles such as fatty oils, for example sesame oil, or synthetic fatty acid esters, for example ethyl oleate or triglycerides, are used, or aqueous injection suspensions which contain viscosity-increasing substances, for example sodium carboxymethylcellulose, sorbitol and/or dextran and, where appropriate, also stabilizers.

The dosage of the active compound depends on the warm-blooded species, the age and the individual condition as well as the mode of administration. In the normal case for a warm-blooded animal weighing about 75 kg the estimated approximate daily dose on oral administration is about 150 mg to about 1500 mg, advantageously in several equal part-doses.

The examples which follow illustrate the invention described above; however, they are not intended to restrict the scope of the latter in any way. Temperatures are stated in degrees celsius. Temperatures are given in degrees Centigrade. If not mentioned otherwise, all evaporations are performed under reduced pressure, preferably between about 15 and 100 mm Hg. The structure of final products, intermediates and starting materials is confirmed by standard analytical methods, e.g. microanalysis and spectroscopic characteristics (e.g. MS, IR, NMR). Abbreviations used are those conventional in the art.

The chemical name of the basic frameworks from which the rifamycin derivatives of the present invention are derived is as follows:

1-deoxy-15-deoxo-1,15-oxy-rifamycin (A) [$R_3$ and $R_3'$ denote a common bond] and 11-hydroxy-11,15-dideoxo-1-deoxy-1,15-oxy-rifamycin (B) [$R_3$ and $R_3'$ each denote hydrogen] can be represented as follows:

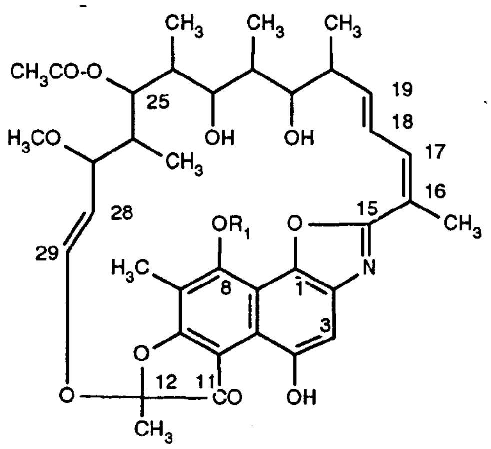

(A)

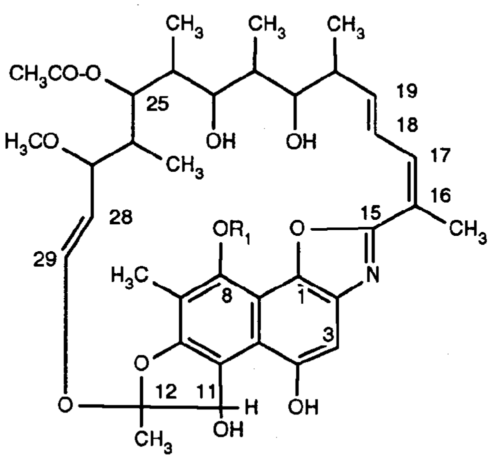

(B)

The stereochemical configurations corresponding to compounds of formula Ia (derived from rifamycin SV) are e.g. as follows:

(a) 1-deoxy-15-deoxo-1,15-oxy-rifamycin (A') [$R_3$ and $R_3'$ denote a common bond], and

(b) 16, 17, 18, 19-tetrahydro-11-hydroxy- 11,15-dideoxo-1-deoxy-1, 15-oxy-rifamycin (B) [$R_3$ an $R_3'$ each denote hydrogen].

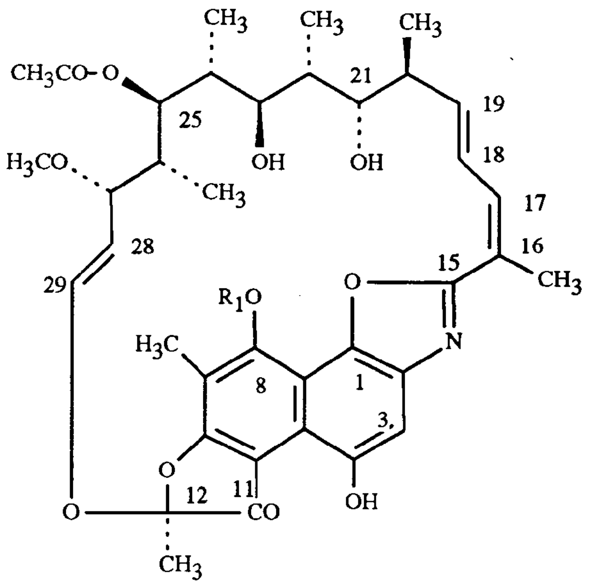

(A')

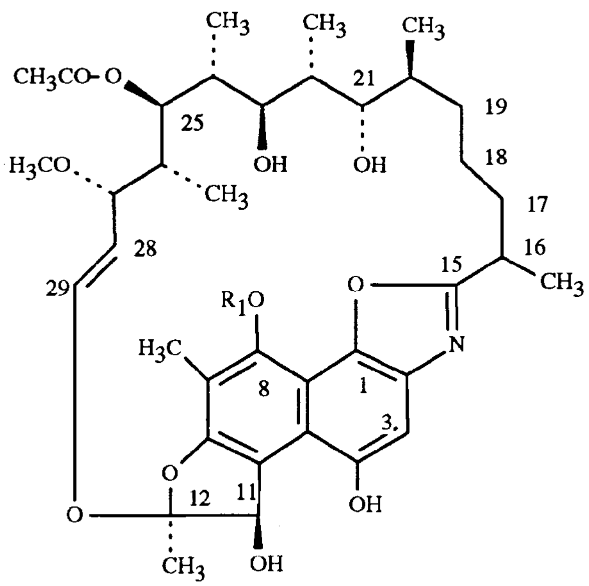

(B')

The stereochemical configuration of the products of the examples is, depending on the involved structure, assigned as depicted in formula A' or B', unless otherwise specified.

The configurations are assigned as in rifamycin SV. Furthermore, in 16,17-dihydro derivatives the 16-methyl group is assigned α, and in 11-hydroxy derivatives the 11-hydroxy group is assigned β.

Example 1:

A mixture of 3-[4-(1-adamantylmethyl)-piperazin-1-yl]-8-O-pivaloyl-1-deoxy-15-deoxo-1,15-oxy-rifamycin (0.59 g), glacial acetic acid (0.1 ml), acetic anhydride (4.5 ml), ethyl acetate (15 ml) and platinum oxide (110 mg) is hydrogenated at standard temperature and pressure for 3.5 days. It is filtered thoursough diatomaceous earth, diluted with ethyl acetate, washed with saturated sodium bicarbonate, then with brine, dried over magnesium sulphate and concentrated to dryness at reduced pressure. The residue is chromatographed through silica gel with 1:3 ethyl acetate-hexane and the major material isolated as pure 11-acetoxy-4-O-acetyl-3-[4-(1-adamantylmethyl)-piperazin-1-yl]-16, 17, 18, 19,28,29-hexahydro- 11,15-dideoxo-1-deoxy-8-O-pivaloyl-1,15-oxy-rifamycin, mp 143- 146°.

The starting material can be prepared, for example, in the following manner: Bromine (0.63 ml) is added slowly to a mixture of rifamycin S (5 g), ethyl acetate (60 ml) and pyridine ( 1.74 ml) at -10° under nitrogen with stirring. After 2 hours at -5°, the solution is washed with 5% cold aqueous sodium thiosulphate (20 x 2 ml), then

with cold water, dried over magnesium sulphate and filtered. The solution is treated at room temperature with 1-(1-adamantylmethyl)-piperazine (1.68 g, prepared and described in Chem. Abstracts 74, P141906q) and triethylamine (1.2 ml). After 18.5 hours, the mixture is concentrated to dryness at reduced pressure to a dark residue, redissolved in ethyl acetate ( 100 ml), washed with saturated sodium bicarbonate solution, then with brine, dried over magnesium sulphate and concentrated to dryness at reduced pressure. The residue is flash chromatographed over silica with 3% methanol in methylene chloride as eluent to purify the desired 3-[4-(1-adamantylmethyl)piperazin-1-yl-rifamycin, mp 200-210°. This solid (2.0 g) in methanol (80 ml) is treated with a solution of sodium ascorbate (3 g) in water ( 15 ml) and methanol (25 ml) dropwise over 40 minutes. It is concentrated to ca. 30 ml at reduced pressure, extracted with methylene chloride and the organic layer is dried over magnesium sulphate and concentrated to dryness at reduced pressure. The residual orange solid is dissolved in ethyl acetate, cooled to -20° and treated with 4-dimethylaminopyridine (13 mg) and pivaloyl chloride (0.5 ml). Triethylamine (0.7 ml) is added slowly at -20° and stirred 23 hours at - 10°. It is treated with ethyl acetate ( 15 ml), pivaloyl chloride (0.25 ml) and triethylamine (0.35 ml) for 1 hour to remove all starting material. The mixture is quenched in water (30 ml) and stirred 10 minutes at ice temperature. The organic layer is washed with saturated sodium bicarbonate (30 x 2 ml), water (30 ml) and then brine and dried over magnesium sulphate to afford an orange solid ( 1. 8 g). This is dissolved in 2-methoxyethanol (70 ml) and heated at reflux under nitrogen for 70 minutes. The mixture is concentrated to dryness at reduced pressure to afford the starting material for Example 1 as a red-violet solid, mp 130- 136°.

Example 2:

To a stirring solution of 3-[4-(1-adamantylmethyl)-piperazin-1-yl]-8-O-pivaloyl-1-deoxy-15-deoxo-1,15-oxy-rifamycin (1.016 g), tetrahydrofuran (30 ml) and water (4 ml) under argon is added sodium borohydride (50 mg) at room temperature. After 2 minutes the reaction is quenched with methanol (4 ml) and concentrated to dryness at reduced pressure. The residual solid, 3- [4-(1-adamantylmethyl)-piperazin-1-yl]-11 -hydroxy-8-O-pivaloyl-1-deoxy-11,15-dideoxo-1,15-oxy-rifamycin, is dissolved in acetone ( 12 ml) and treated under argon with potassium carbonate (0.59 g) followed by dimethyl sulphate (0.22 ml). It is stirred overnight at room temperature, then concentrated to dryness at reduced pressure, taken up in methylene chloride (40 ml), washed with water, then with brine and dried over magnesium sulphate. After removal of solvent at reduced pressure, the residue is flash chromatographed over silica with ether-hexane as eluent (1:1, then later 3: 2). The desired 3- [4-(1-adamantyl-methyl)-piperazin-1-yl]-11-hydroxy-4-O-methyl-8-O-pivaloyl-1-deoxy-11, 15-dideoxo1,15-oxy-rifamycin is obtained as a light colored solid, mp 136- 140°.

Example 3:

The product from Example 2 (0.49 g) is dissolved in ethanol (25 ml), treated with 10% palladium on charcoal (0.25 g) and hydrogenated at 50 psi over 5 days. The catalyst is removed by filtration and the solvent removed at reduced pressure. The residue is dissolved in methylene chloride (50 ml), washed with water (35 ml), saturated sodium bicarbonate and then with brine and dried over magnesium sulphate. Removal of the solvent at reduced pressure gives 3-[4-(1-adamantylmethyl)-piperazin-1-yl]- 11-hydroxy-4-O-methyl-8-O-pivaloyl-16,17,18,19-tetrahydro-1-deoxy-11,15-dideoxo-1,15-oxy-rifamycin, mp 143- 147°.

Example 4:

Capsules containing 250 mg of active compound, for example the compound of the formula I in which the structural elements $-A_1-A_2-$, $-A_3-A_4-$ and $-A_5-A_6-$ each denote vinylene; X represents

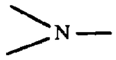 ;

A denotes methylene, $R_1$ denotes pivaloyl; $R_2$ denotes methyl; $R_3$, $R_3'$ and $R_7$ each denote hydrogen; $R_4$ denotes 1-adamantyl; and $R_5$ is acetyl; can be prepared as follows:

Composition (for 1000 capsules):

| | |
|---|---|
| Active Compound | 250.0 g |
| Maize starch | 50.0 g |
| Polyvinylpyrrolidone | 15.0 g |
| Magnesium stearate | 5.0 g |

Ethanol q.s.

The active compound and the maizte starch are mixed and moistened with a solution of polyvinylpyrrolidone in 50 g of ethanol. The moist composition is forced through a screen with a mesh width of 3 mm and dried at 45°. The dry granules are screened through a screen with a mesh width of 1 mm and mixed with 5 g of magnesium stearate. The mixture is dispensed in 0.320 g portions into size 0 two-piece capsules.

It is also possible in an analogous manner to use the other compounds prepared as in the preceding examples as active compound component.

## Claims

1. A rifamycin SV derivative of the formula

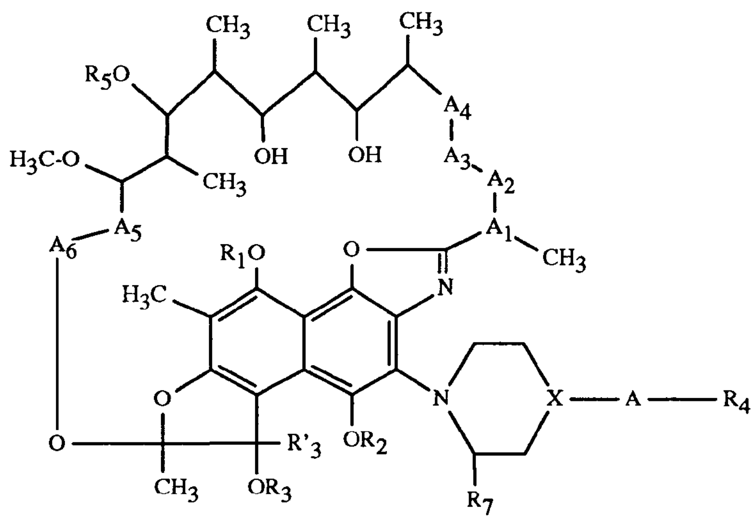

(I)

or a salt thereof, in which the structural elements $-A_1-A_2-$, $-A_3-A_4-$ and $-A_5-A_6-$ each denote ethylene or vinylene or the elements $-A_1-A_2-$ and $-A_3-A_4-$ each denote ethylene and $-A_5-A_6-$denotes vinylene; X represents

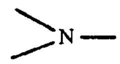

or

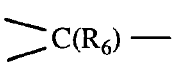

and $R_6$ denotes hydrogen or alkyl; A denotes a bond, a bivalent aliphatic hydrocarbon radical or, if X represents

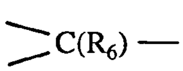

,

represents the structural element $-X_1-A_1-$ in which $X_1$ denotes -O-, $-S(O)_n-$ or $-N(R_6)-$ n being 0, 1 or 2 and $R_6$ being hydrogen or alkyl and in which $A_1$ denotes a bivalent aliphatic hydrocarbon radical; $R_1$ denotes hydrogen or acyl; $R_2$ denotes acyl or an aliphatic radical; and $R_3$ and $R_3'$ represent a common bond; or $R_3$ denotes hydrogen, acyl or an aliphatic radical, and $R_3'$ is hydrogen or an aliphatic radical; $R_4$ denotes optionally substituted bicycloheptyl, bicycloheptenyl or adamantyl; $R_6$ denotes hydrogen or acetyl; $R_7$ denotes hydrogen or alkyl.

2. A compound according to claim 1 of the formula Ia

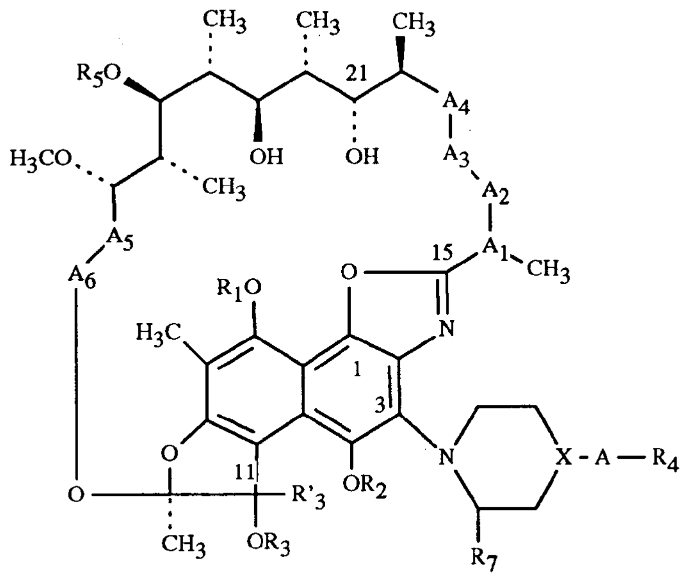

(Ia)

or a salt thereof, in which the structural elements $-A_1-A_2-$, $-A_3-A_4-$ and $-A_5-A_6-$ , the variables X, A, $R_1$, $R_2$, $R_3$, $R_3'$, $R_4$, $R_5$, and $R_7$ have the meanings given in claim 1.

3. A compound according to claim 1 or 2 of the formula I or Ia or a salt thereof, in which the structural elements $-A_1-A_2-$, $-A_3-A_4-$ and $-A_5-A_5-$ have the meanings given; X represents

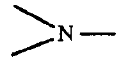

or

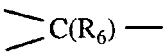

and $R_5$ denotes hydrogen or $C_1$-$C_7$alkyl; A denotes denotes a bond, $C_1$-$C_7$alkylene, $C_3$-$C_7$alkenylene or $C_3$-$C_7$alkynylene or, if X represents

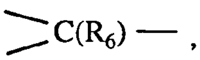 ,

represents the structural element $-X_1-A_1-$ in which $X_1$ denotes -O-, $S(O)_n$- or $-N(R_6)$- n being 0, 1 or 2 and $R_6$ being hydrogen or $C_1$-$C_7$alkyl and in which $A_1$ denotes $C_1C_7$alkylene, $C_3$-$C_7$alkenylene or $C_3$-$C_7$alkynylene; $R_1$ denotes hydrogen, $C_2$-$C_8$alkanoyl, carboxy-$C_2$-$C_8$alkanoyl, $C_1$-$C_7$alkoxycarbonyl- or $C_1$-$C_7$alkoxy-$C_1$-$C_7$alkoxy-carbonyl-$C_2$-$C_8$alkanoyl, carbamoyl-$C_2$-$C_8$alkanoyl, in which carbamoyl is optionally mono- or disubstituted by a radical selected from $C_1$-$C_7$alkyl and phenyl-$C_1$-$C_7$alkyl, denotes halogeno-$C_2$-$C_8$alkanoyl, phenyl- or naphthyl-$C_2$-$C_8$alkanoyl, benzoyl, naphthoyl, 5-or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or denotes $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkanesulfonyl or benzene- or naphthylsulfonyl; $R_2$ denotes $C_2$-$C_8$alkanoyl, carboxy-$C_2$-$C_8$alkanoyl, $C_1$-$C_7$alkoxycarbonyl- or $C_1$-$C_7$alkoxy-$C_1$-$C_7$alkoxy-carbonyl-$C_2$-$C_8$alkanoyl, carbamoyl-$C_2$-$C_8$alkanoyl, in which carbamoyl is optionally mono- or disubstituted by a radical selected from $C_1$-$C_7$alkyl and phenyl-$C_1$-$C_7$alkyl, denotes halogeno-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or denotes $C_1$-$C_7$alkanesulfonyl, halogeno-$C_1$-$C_7$alkanesulfonyl, phenyl- or naphthyl-$C_1$-$C_7$alkanesulfonyl,
$C_3$-$C_7$cycloalkanesulfonyl or benzene- or naphthylsulfonyl, or denotes $C_1$-$C_7$alkyl or $C_3$-$C_7$alkenyl which are optionally substituted by a radical selected from $C_3$-$C_7$cycloalkyl, phenyl, naphthyl, hydroxyl, $C_1$-$C_7$alkoxy, hydroxy-$C_1$-$C_7$alkoxy, $C_1$-$C_7$alkoxy-$C_1$-$C_7$alkoxy, phenyl-$C_1$-$C_7$alkoxy, phenyl-$C_1$-$C_7$alkoxy-$C_1$-$C_7$alkoxy, amino which is optionally substituted by a substituent selected from $C_1$-$C_7$alkyl, phenyl-$C_1$-$C_7$alkyl and phenyl, carboxy, $C_1$-$C_7$alkoxycarbonyl, phenyl-$C_1$-$C_7$alkoxycarbonyl or $C_1$-$C_7$alkoxy-$C_1$-$C_7$alkoxycar-

bonyl, carbamoyl which is optionally substituted by a substituent selected from $C_1$-$C_7$alkyl, phenyl-$C_1$-$C_7$alkyl and phenyl; $R_3$ and $R_3'$ represent a common bond; or $R_3$ denotes hydrogen, $C_2$-$C_8$alkanoyl, carboxy-$C_2$-$C_8$alkanoyl, $C_1$-$C_7$alkoxycarbonyl- or $C_1$-$C_7$alkoxy-$C_1$-$C_7$alkoxy-carbonyl-$C_2$-$C_8$alkanoyl, carbamoyl-$C_2$-$C_8$alkanoyl, in which carbamoyl is optionally mono- or disubstituted by a radical selected from $C_1$-$C_7$alkyl and phenyl-$C_1$-$C_7$alkyl, denotes halogeno-$C_2$-$C_8$alkanoyl, phenyl- or naphthyl-$C_2$-$C_8$alkanoyl, benzoyl, naphthoyl, 5- or 6-membered monocyclic monoaza-, monooxa- or monothiaaroyl, $C_1$-$C_7$alkoxycarbonyl, phenyl- or naphthyl-$C_1$-$C_7$alkoxy-carbonyl, aminocarbonyl which is unsubstituted or mono- or di-substituted by $C_1$-$C_7$alkyl, or denotes $C_1$-$C_7$alkanesulfonyl, halogeno-$C_1$-$C_7$alkanesulfonyl, phenyl- or naphthyl-$C_1$-$C_7$alkanesulfonyl, $C_3$-$C_7$cycloalkanesulfonyl or benzene- or naphthylsulfonyl, or denotes $C_1$-$C_7$alkyl or $C_3$-$C_7$alkenyl which are optionally substituted by a radical selected from $C_3$-$C_7$cycloalkyl, phenyl, naphthyl, hydroxyl, $C_1$-$C_7$alkoxy, hydroxy-$C_1$-$C_7$alkoxy, $C_1$-$C_7$alkoxy-$C_1$-$C_7$alkoxy, phenyl-$C_1$-$C_7$alkoxy, phenyl-$C_1$-$C_7$alkoxy-$C_1$-$C_7$alkoxy, amino which is optionally substituted by a substituent selected from $C_1$-$C_7$alkyl, phenyl-$C_1$-$C_7$alkyl and phenyl, carboxy, $C_1$-$C_7$alkoxycarbonyl, phenyl-$C_1$-$C_7$alkoxycarbonyl or $C_1$-$C_7$alkoxy-$C_1$-$C_7$alkoxycarbonyl, carbamoyl which is optionally substituted by a substituent selected from $C_1$-$C_7$alkyl, phenyl-$C_1$-$C_7$alkyl and phenyl; and $R_3'$ denotes hydrogen, $C_1$-$C_7$alkyl or $C_3$-$C_7$alkenyl which are optionally substituted by a radical selected from $C_3$-$C_7$cycloalkyl, phenyl, naphthyl, hydroxyl, $C_1$-$C_7$alkoxy, hydroxy-$C_1$-$C_7$alkoxy, $C_1$-$C_7$alkoxy-$C_1$-$C_7$alkoxy, phenyl-$C_1$-$C_7$alkoxy, phenyl-$C_1$-$C_7$alkoxy-$C_1$-$C_7$alkoxy, amino which is optionally substituted by a substituent selected from $C_1$-$C_7$alkyl, phenyl-$C_1$-$C_7$alkyl and phenyl, carboxy, $C_1$-$C_7$alkoxycarbonyl, phenyl-$C_1$-$C_7$alkoxycarbonyl or $C_1$-$C_7$alkoxy-$C_1$-$C_7$alkoxycarbonyl, carbamoyl which is optionally substituted by a substituent selected from $C_1$-$C_7$alkyl, phenyl-$C_1$-$C_7$alkyl and phenyl; $R_4$ denotes bicycloheptyl, bicycloheptenyl or adamantyl each of which may be substituted by $C_1$-$C_7$alkyl; $R_5$ denotes hydrogen or acetyl; $R_7$ denotes hydrogen or $C_1$-$C_7$alkyl; where the (hetero-)aromatic radicals in each case are unsubstituted or substituted one or more times by a substituent selected from the group consisting of halogen, $C_1$-$C_7$alkyl, $C_1$-$C_7$alkoxy, hydroxyl, $C_2$-$C_8$alkanoyloxy, trifluoromethyl and nitro.

4. A compound according to claim 1 or 2 of the formula I or Ia or a salt thereof, in which the structural elements $-A_1$-$A_2$-, $-A_3$-$A_4$- and $-A_5$-$A_6$- have the meanings given; X represents

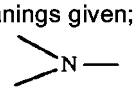

or

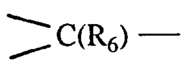

and $R_6$ denotes hydrogen; A denotes $C_1$-$C_4$alkylene, in particular, methylene; $R_1$ denotes hydrogen or branched $C_3$-$C_6$alkanoyl, in particular, pivaloyl; $R_2$ denotes $C_2$-$C_6$alkanoyl, or denotes $C_1$-$C_4$alkyl which is optionally substituted by a radical selected from $C_3$-$C_7$cycloalkyl, phenyl, hydroxyl, $C_1$-$C_4$alkoxy, hydroxy-$C_1$-$C_4$alkoxy, $C_1$-$C_4$alkoxy-$C_1$-$C_4$alkoxy, phenyl-$C_1$-$C_4$alkoxy, phenyl-$C_1$-$C_4$alkoxy-$C_1$-$C_4$alkoxy, carboxy, $C_1$-$C_4$alkoxycarbonyl, phenyl-$C_1$-$C_4$alkoxycarbonyl, and $C_1$-$C_4$alkoxy-$C_1$-$C_4$alkoxycarbonyl; $R_3$ denotes hydrogen, $C_2$-$C_6$alkanoyl, carboxy-$C_2$-$C_6$alkanoyl, $C_1$-$C_4$alkoxycarbonyl- or $C_1$-$C_4$alkoxy-$C_1$-$C_4$alkoxy-carbonyl-$C_2$-$C_6$alkanoyl, or denotes $C_1$-$C_4$alkyl which is optionally substituted by a radical selected from $C_1$-$C_4$alkoxy, $C_1$-$C_4$alkoxy-$C_1$-$C_4$alkoxy, phenyl-$C_1$-$C_4$alkoxy, carboxy, and $C_1$-$C_4$alkoxycarbonyl; and $R_3'$ denotes hydrogen or $C_1$-$C_4$alkyl; $R_4$ denotes bicycloheptyl, bicycloheptenyl or, in particular, adamantyl each of which may be substituted by $C_1$-$C_4$alkyl; $R_5$ denotes hydrogen or acetyl; $R_7$ denotes hydrogen or $C_1$-$C_4$alkyl.

5. A compound according to claim 1 or 2 of the formula I or Ia or a salt thereof, in which the structural elements $-A_1$ $-A_2$-, $-A_3$-$A_4$- and $-A_5$-$A_6$- have the meanings given; X represents

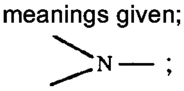 ;

A denotes methylene; $R_1$ denotes pivaloyl; $R_2$ denotes $C_2$-$C_6$alkanoyl, or denotes $C_1$-$C_4$alkyl which is optionally substituted by a radical selected from $C_1$-$C_4$alkoxy, $C_1$-$C_4$alkoxy-$C_1$-$C_4$alkoxy; $R_3$ denotes hydrogen, $C_2$-$C_6$alkanoyl, carboxy-$C_2$-$C_6$alkanoyl, or $C_1$-$C_4$alkoxycarbonyl-$C_2$-$C_6$alkanoyl, or denotes $C_1$-$C_4$alkyl which is optionally substituted by a radical selected from $C_1$-$C_4$alkoxy, $C_1$-$C_4$alkoxy-$C_1$-$C_4$alkoxy, carboxy, and $C_1$-$C_4$alkoxycarbonyl; and $R_3'$ denotes hydrogen or $C_1$-$C_4$alkyl; $R_4$ denotes adamantyl; $R_5$ denotes acetyl; $R_7$ denotes hydrogen or $C_1$-$C_4$alkyl.

6. A compound according to claim 1 or 2 of the formula I or Ia or a salt thereof, in which the structural elements $-A_1-A_2-$, $-A_3-A_4-$ and $-A_5-A_6-$ have the meanings given; X represents

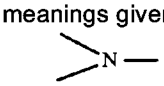 ;

A denotes methylene; $R_1$ denotes pivaloyl; $R_2$ denotes $C_1$-$C_4$alkyl which is optionally substituted by a radical selected from $C_1$-$C_4$alkoxy and $C_1$-$C_4$alkoxy-$C_1$-$C_4$alkoxy; $R_3$ and $R_3'$ denote hydrogen; $R_4$ denotes adamantyl; $R_5$ denotes acetyl; $R_7$ denotes hydrogen.

7. A compound according to claim 1 or 2 of the formula I or Ia or a salt thereof, in which the structural elements $-A_1-A_2-$, $-A_3-A_4-$ and $-A_5-A_6-$ have the meanings given; X represents

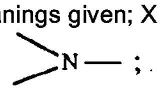 ;.

A denotes methylene; $R_1$ denotes pivaloyl; $R_2$ denotes $C_1$-$C_4$alky; $R_3$ and $R_3'$ denote hydrogen; $R_4$ denotes adamantyl; $R_5$ denotes acetyl; $R_7$ denotes hydrogen.

8. A compound according to claim 1 or 2 being 11-acetoxy-4-O-acetyl-3-[4-(adamantyl-methyl)-piperazin-1-yl]- 16, 17, 18, 19,28,29-hexahydro-11,15-dideoxo- 1-deoxy-8-pivaloyl-1,15-oxy-rifamycin or a salt thereof; 3-[4-(1-adamantylmethyl)-piperazin-1-yl]-11-hydroxy-4-O-methyl-8-O-pivaloyl-1-deoxy-1 1,15-dideoxo-1,15-oxy-rifamycin or a salt thereof; or 3-[4-(1-adamantylmethyl)-piperazin-1-yl]-11-hydroxy-4-O-methyl-8-O-pivaloyl-16, 17, 18, 19-tetrahydro-1-deoxy-11,15-dideoxo-1,15,oxy-rifamycin or a salt thereof.

9. A pharmaceutical composition, comprising a therapeutically effective amount of a compound according to any one of claims 1 to 8 or a pharmaceutically acceptable salt thereof with pharmaceutically acceptable carriers.

10. Use of a compound of the formula I or Ia or a pharmaceutically acceptable salt thereof for the manufacture of a pharmaceutical composition for use for the treatment of hyperlipidaemias and arteriosclerosis.

European Patent Office

# EUROPEAN SEARCH REPORT

Application Number

EP 91810675.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP - A - 0 434 621 (CIBA-GEIGY AG) * Abstract * -- | 1,9,10 | C 07 D 498/18<br>A 61 K 31/42<br>//(C 07 D 498/18<br>C 07 D 323:00<br>C 07 D 307:00<br>C 07 D 263:00) |
| D,A | EP - A - 0 314 624 (CIBA-GEIGY AG) * Abstract * -- | 1,9,10 | |
| A | EP - A - 0 303 571 (CIBA-GEIGY AG) * Abstract * -- | 1,9,10 | |
| A | EP - B - 0 244 398 (CIBA-GEIGY AG) * Claims * -- | 1,9,10 | |
| A | EP - A - 0 213 935 (AMERICAN HOME PRODUCTS) * Abstract * ---- | 1,9,10 | |

TECHNICAL FIELDS SEARCHED (Int. Cl.5)

C 07 D 498/00
A 61 K 31/00

The present search report has been drawn up for all claims

| Place of search | Date of completion of the search | Examiner |
|---|---|---|
| VIENNA | 11-12-1991 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)